# EUROPEAN PATENT APPLICATION

(11) **EP 3 269 313 A1**
(43) Date of publication of application: **17.01.2018**
(21) Application number: 17180570.8
(22) Date of filing: 10.07.2017
(51) Int. Cl.: A61B 17/128, A61B 90/00, A61B 17/00

(54) **ENDOSCOPIC REPOSABLE SURGICAL CLIP APPLIER**

(30) Priority: 11.07.2016 US 201662360498 P; 26.05.2017 US 201715606366
(71) Applicant: Covidien LP, Mansfield, MA 02048 (US)
(72) Inventor: BARIL, Jacob, White Plains, NY New York 10605 (US)
(74) Representative: Maschio, Antonio

(57) **Abstract**

An endoscopic reposable surgical clip applier is provided and includes a handle assembly and an endoscopic assembly. The endoscopic assembly is selectively connectable to and in mechanical communication with the handle assembly and includes a shaft assembly. The shaft assembly includes a pair of jaws pivotably and fixedly supported in, and extending from a distal portion of the shaft assembly, a spindle assembly supported within the endoscopic assembly, and a lockout mechanism fixedly supported on the pair of jaws. A distal end of the spindle is operatively engaged with the pair of jaws to effectuate an opening and a closing of the pair of jaws upon an axial translation of the spindle. The lockout mechanism is in selective engagement with the spindle and includes a first position that enables distal advancement of the spindle and a second position that inhibits distal advancement of the spindle.

## Description

### CROSS-REFERENCE TO RELATED APPLICATIONS

This application claims the benefit of and priority to U.S. Provisional Patent Application No. 62/360,498 filed July 11, 2016, the entire disclosure of which is incorporated by reference herein.

### BACKGROUND

### Technical Field

The technical field relates to surgical clip appliers. More particularly, the present disclosure relates to endoscopic reposable surgical clip appliers having a reusable handle assembly, at least one reusable shaft assembly, and at least one disposable clip cartridge assembly.

### Description of Related Art

Endoscopic surgical staplers and surgical clip appliers are known in the art and are used for a number of distinct and useful surgical procedures. In the case of a laparoscopic surgical procedure, access to the interior of an abdomen is achieved through narrow tubes or cannulas inserted through a small entrance incision in the skin. Minimally invasive procedures performed elsewhere in the body are often generally referred to as endoscopic procedures. Typically, a tube or cannula device is extended into the patient's body through the entrance incision to provide an access port. The port allows the surgeon to insert a number of different surgical instruments therethrough using a trocar and for performing surgical procedures far removed from the incision.

During a majority of these procedures, the surgeon must often terminate the flow of blood or another fluid through one or more vessels. The surgeon will often use a particular endoscopic surgical clip applier to apply a surgical clip to a blood vessel or another duct to prevent the flow of body fluids therethrough during the procedure.

Endoscopic surgical clip appliers having various sizes (e.g., diameters), that are configured to apply a variety of diverse surgical clips, are known in the art, and which are capable of applying a single or multiple surgical clips during an entry to the body cavity. Such surgical clips are typically fabricated from a biocompatible material and are usually compressed over a vessel. Once applied to the vessel, the compressed surgical clip terminates the flow of fluid therethrough.

Endoscopic surgical clip appliers that are able to apply multiple clips in endoscopic or laparoscopic procedures during a single entry into the body cavity are described in commonly-assigned U.S. Pat. Nos. 5,084,057 and 5,100,420 to Green et al., which are both incorporated by reference in their entirety. Another multiple endoscopic surgical clip applier is disclosed in commonly-assigned U.S. Pat. No. 5,607,436 by Pratt et al., the contents of which is also hereby incorporated by reference herein in its entirety. These devices are typically, though not necessarily, used during a single surgical procedure. U.S. Pat. No. 5,695,502 to Pier et al., the disclosure of which is hereby incorporated by reference herein, discloses a resterilizable endoscopic surgical clip applier. The endoscopic surgical clip applier advances and forms multiple clips during a single insertion into the body cavity. This resterilizable endoscopic surgical clip applier is configured to receive and cooperate with an interchangeable clip magazine so as to advance and form multiple clips during a single entry into a body cavity.

During endoscopic or laparoscopic procedures it may be desirable and/or necessary to use different size surgical clips or different configured surgical clips depending on the underlying tissue or vessels to be ligated. In order to reduce overall costs of an endoscopic surgical clip applier, it is desirable for a single endoscopic surgical clip applier to be loadable with and capable of firing different size surgical clips as needed.

Accordingly, a need exists for endoscopic surgical clip appliers that include reusable handle assemblies, reusable shaft assemblies, and disposable clip cartridge assemblies, with each clip cartridge assembly being loaded with a particularly sized clip (e.g., relatively small, relatively medium, or relatively large).

### SUMMARY

The present disclosure relates to reposable endoscopic surgical clip appliers.

According to an aspect of the present disclosure, a reposable surgical clip applier is provided. The reposable surgical clip applier includes a handle assembly and an endoscopic assembly selectively connectable to and in mechanical communication with the handle assembly. The endoscopic assembly includes a shaft assembly. The shaft assembly includes a pair of jaws pivotably and fixedly supported in, and extending from a distal portion of the shaft assembly, a spindle slidably supported within the endoscopic assembly, and a lockout mechanism fixedly supported on the pair of jaws. The spindle includes a proximal end and a distal end. The distal end of the spindle is operatively engaged with the pair of jaws to effectuate an opening and a closing of the pair of jaws upon an axial translation of the spindle relative to the pair of jaws. The lockout mechanism is in selective engagement with the spindle. The lockout mechanism includes a first position that enables a distal advancement of the spindle and a second position that inhibits distal advancement of the spindle.

In aspects, the shaft assembly may further include a pusher bar slidably supported within the endoscopic assembly.

In embodiments, the shaft assembly may further include a clip follower slidably disposed within the endoscopic assembly and interposed between the pusher bar and the pair of jaws.

In certain aspects, a distal portion of the clip follower may define a window therethrough that is configured to receive a pusher defined on a distal portion of the pusher bar.

In some aspects, a proximal portion of the clip follower may define a wedge extending proximally and outwardly therefrom. The wedge may be configured to selectively engage a portion of the lockout mechanism.

In embodiments, the lockout mechanism may define a distal elongate tab portion and a proximal wedge portion. The distal elongate tab portion is configured to be interposed between the pair of jaws and the spindle. The proximal wedge portion may be configured to selectively engage the wedge of the clip follower as the wedge is advanced over the proximal wedge portion of the lockout mechanism.

In aspects, the lockout mechanism may include a resilient finger that couples the proximal wedge portion to the distal elongate tab portion thereof such that the proximal wedge portion is free to rotate relative to the distal elongate tab portion.

In certain aspects, the spindle may define a window therethrough that is configured to receive the proximal wedge portion of the lockout mechanism, wherein, when the proximal wedge portion of the lockout mechanism is disposed in the window of the spindle, further distal advancement of the spindle is inhibited.

In embodiments, the shaft assembly may define a plurality of shear pins on an interior surface thereof.

In some aspects, the pair of jaws and the lockout mechanism may define a plurality of through holes configured to receive the plurality of shear pins therein.

In aspects, the shear pins may be configured to shear from the shaft assembly when a force exceeding a threshold value is applied to the shear pins.

In certain aspects, the resilient finger may be configured to bias the proximal wedge portion of the lockout mechanism in an upward direction with respect to the distal elongate tab portion of the lockout mechanism.

According to another aspect of the present disclosure, a lockout assembly for use with a reposable surgical clip applier is provided. The lockout assembly includes a clip follower and a lockout mechanism. The clip follower includes a proximal portion and a distal portion. The distal portion of the clip follower is configured to engage a pusher bar of a reposable surgical clip applier. The lockout mechanism includes a distal elongate tab portion, a proximal wedge portion, and a resilient finger interposed between the distal elongate tab portion and the proximal wedge portion to couple the distal elongate tab portion to the proximal wedge portion. The proximal portion of the clip follower is configured to selectively engage the proximal wedge portion of the lockout mechanism such that when the proximal wedge portion of the lockout mechanism is engaged by the proximal portion of the clip follower, the proximal wedge portion is configured to inhibit closure of a pair of jaws of the reposable surgical clip applier.

In aspects, the distal portion of the clip follower may define a window therethrough that may be configured to receive a portion of a pusher of a reposable surgical clip applier.

In certain aspects, the proximal portion of the clip follower may define a wedge extending proximally and outwardly therefrom that may be configured to selectively engage the proximal wedge portion of the lockout mechanism as the wedge of the clip follower is advanced over the proximal wedge portion of the lockout mechanism.

In some aspects, when the proximal wedge portion is in a first position, a pair of jaws of a reposable surgical clip applier may be permitted to close, and when the proximal wedge portion is in a second position, a pair of jaws of a reposable surgical clip applier may be inhibited from closing.

In certain aspects, the proximal wedge portion may define a proximal face that is configured to engage a spindle of a reposable surgical clip applier to inhibit closure of a pair of jaws of a reposable surgical clip applier.

In aspects, the lockout mechanism may define a plurality of bores that are configured to receive a corresponding plurality of shear pins disposed on a reposable surgical clip applier.

In some aspects, the proximal wedge portion of the lockout mechanism may define a U-shaped configuration.

In certain aspects, the lockout mechanism may be maintained in fixed relation to a pair of jaws of a reposable surgical clip applier.

### BRIEF DESCRIPTION OF THE DRAWINGS

A particular embodiment of a surgical clip applier is disclosed herein with reference to the drawings wherein:
FIG. 1 is a perspective view of a reposable endoscopic surgical clip applier, according to the present disclosure including a reusable handle assembly, and a first endoscopic assembly and a second endoscopic assembly each selectively connectable to the handle assembly;
FIG. 2 is perspective view of the reposable endoscopic surgical clip applier including the reusable handle assembly and the first endoscopic assembly connected thereto;
FIG. 3 is a perspective view of the handle assembly with at least a housing half-section removed therefrom;
FIG. 4 is a perspective view, with parts separated, of the handle assembly of FIGS. 1-3;
FIG. 5 is an enlarged perspective view of the indicated area of detail of FIG. 4, illustrating a pawl switch and a pawl actuator of the handle assembly of FIG. 1;
FIG. 6 is a further perspective view of the pawl switch of FIG. 5;
FIG. 7 is a further perspective view of the pawl actuator of FIG. 5;
FIGS. 8-9 are various perspective views of the pawl switch and the pawl actuator of the handle assembly, shown in operation with the pawl switch in an un-actuated condition and the pawl actuator engaged with a pawl of a ratchet assembly;
FIG. 10 is a top plan view of the pawl switch and the pawl actuator of the handle assembly, shown in operation with the pawl switch in the un-actuated condition and the pawl actuator engaged from the pawl of the ratchet assembly;
FIG. 11 is a transverse, cross-sectional view of the handle assembly of FIG. 1 as taken through 11-11 of FIG. 1, illustrating the pawl switch in an actuated condition;
FIGS. 12-13 are various perspective views of the pawl switch and the pawl actuator of the handle assembly, shown in operation with the pawl switch in the actuated condition and the pawl actuator disengaged from the pawl of the ratchet assembly;
FIG. 14 is a top plan view of the pawl switch and the pawl actuator of the handle assembly, shown in operation with the pawl switch in the actuated condition and the pawl actuator disengaged from the pawl of the ratchet assembly;
FIG. 15 is a perspective view, with parts separated, of the first endoscopic assembly of FIG. 1;
FIG. 16 is a top, plan view of the first endoscopic assembly of FIGS. 1 and 15;
FIG. 17 is a transverse, cross-sectional view of the first endoscopic assembly of FIGS. 1 and 15-16, as taken through 17-17 of FIG. 16;
FIG. 18 is a perspective view illustrating an initial connection of the handle assembly and the first endoscopic assembly;
FIG. 19 is a longitudinal, transverse cross-sectional view illustrating the initial connection of the handle assembly and the first endoscopic assembly;
FIG. 20 is an enlarged view of the indicated area of detail of FIG. 19;
FIG. 21 is a longitudinal, transverse cross-sectional view illustrating a complete connection of the handle assembly and the first endoscopic assembly;
FIG. 22 is an enlarged view of the indicated area of detail of FIG. 21;
FIG. 23 is a longitudinal, transverse cross-sectional view illustrating an initial actuation of the handle assembly with the first endoscopic assembly connected thereto;
FIG. 24 is an enlarged view of the indicated area of detail of FIG. 23;
FIG. 25 is a longitudinal, transverse cross-sectional view illustrating a complete actuation of the handle assembly with the first endoscopic assembly connected thereto;
FIG. 26 is perspective view of the reposable endoscopic surgical clip applier including the reusable handle assembly and the second endoscopic assembly connected thereto;
FIG. 27 is a perspective view, with parts separated, of the second endoscopic assembly of FIGS. 1 and 26;
FIG. 28 is a perspective view, with parts separated, of a shaft assembly of the second endoscopic assembly;
FIG. 29 is a perspective view of the distal end of the shaft assembly of the second endoscopic assembly with an outer tube removed therefrom;
FIG. 30 is an enlarged view of the indicated area of detail of FIG. 29;
FIG. 31 is an enlarged view of the indicated area of detail of FIG. 29;
FIG. 32 is a perspective view of the distal end of the shaft assembly of the second endoscopic assembly with the outer tube and a pusher bar removed therefrom;
FIG. 33 is an enlarged view of the indicated area of detail of FIG. 32;
FIG. 34 is an enlarged view of the indicated area of detail of FIG. 32;
FIG. 35 is a perspective view of the distal end of the shaft assembly of the second endoscopic assembly with the outer tube, the pusher bar and a clip channel removed therefrom;
FIG. 36 is an enlarged view of the indicated area of detail of FIG. 35;
FIG. 37 is an enlarged view of the indicated area of detail of FIG. 35;
FIG. 38 is a perspective view of the distal end of the shaft assembly of the second endoscopic assembly with the outer tube, the pusher bar, the clip channel and a pair of jaws and a filler component removed therefrom;
FIG. 39 is a perspective view of the distal end of the shaft assembly of the second endoscopic assembly with the outer tube, the pusher bar, the clip channel, the pair of jaws, the filler component, and a wedge plate removed therefrom;
FIG. 40 is a longitudinal, transverse cross-sectional view illustrating a complete connection of the handle assembly and the second endoscopic assembly, prior to actuation of a trigger of the handle assembly;
FIG. 41 is a longitudinal, transverse cross-sectional view illustrating a complete actuation of the handle assembly with the second endoscopic assembly connected thereto;
FIG. 42 is a perspective view, of a third endoscopic assembly, for use with the reposable endoscopic surgical clip applier of FIG. 26 in accordance with the present disclosure, shown with parts separated;
FIG. 43 is a side, cross-sectional view, of the shaft assembly of the third endoscopic assembly of FIG. 42, shown with parts separated;
FIG. 44 is perspective view a lockout mechanism of the shaft assembly of FIG. 43;
FIG. 45 is a perspective view of a clip follower of the shaft assembly of FIG. 43;
FIG. 46 is a perspective view of an outer tube of the shaft assembly of FIG. 43;
FIG. 47 is a perspective view of a pusher bar of the shaft assembly of FIG. 43 received within the clip follower of FIG. 45;
FIG. 48 is a perspective view of the shaft assembly of FIG. 43;
FIG. 49 is a side, cross-sectional view, of the shaft assembly of FIG. 43, shown with the lockout mechanism in a first position;
FIG. 50 is a side, cross-sectional view, of the shaft assembly of FIG. 43, shown with the spindle assembly advanced in a distal position;
FIG. 51 is a side, cross-sectional view, of the shaft assembly of FIG. 43, shown with the lockout mechanism in a second position; and
FIG. 52 is a schematic illustration of a robotic surgical system configured for use in accordance with the present disclosure.

### DETAILED DESCRIPTION OF EMBODIMENTS

Embodiments of reposable endoscopic surgical clip appliers, in accordance with the present disclosure, will now be described in detail with reference to the drawing figures wherein like reference numerals identify similar or identical structural elements. As shown in the drawings and described throughout the following description, as is traditional when referring to relative positioning on a surgical instrument, the term "proximal" refers to the end of the apparatus which is closer to the user and the term "distal" refers to the end of the apparatus which is further away from the user.

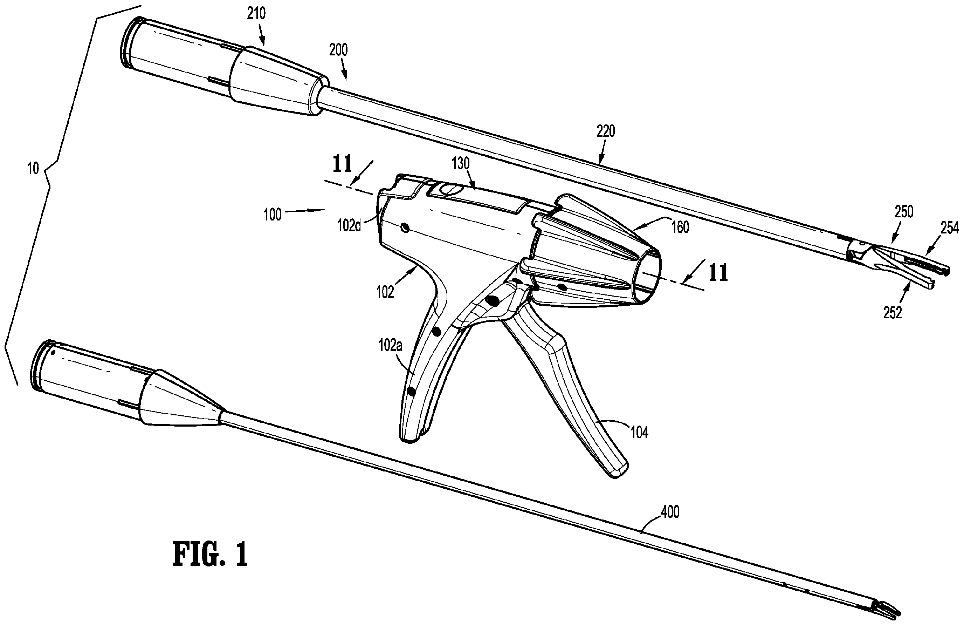

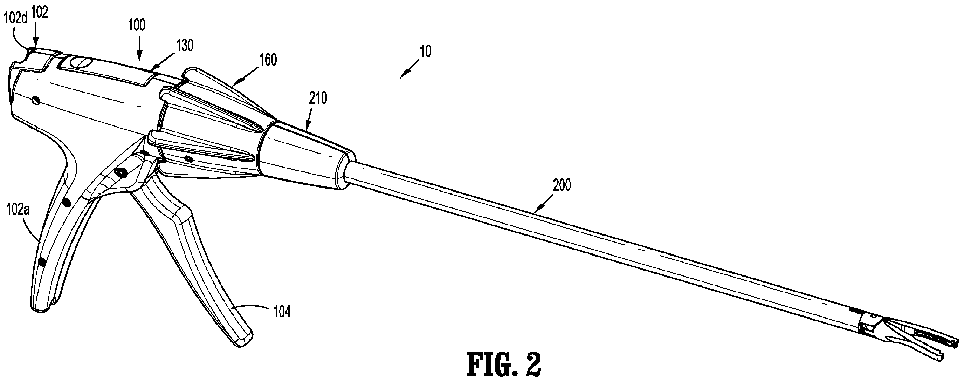

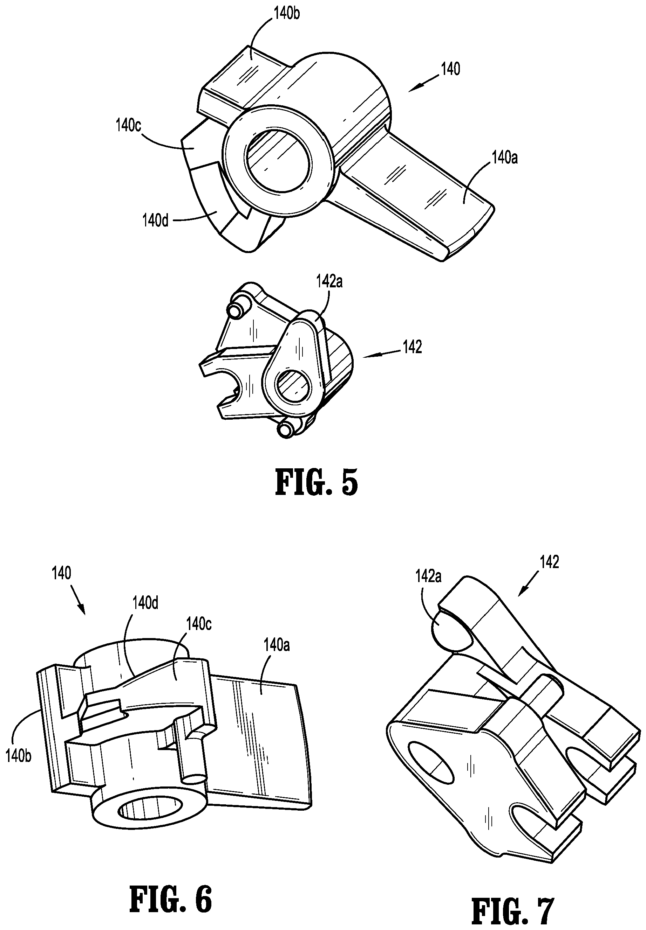

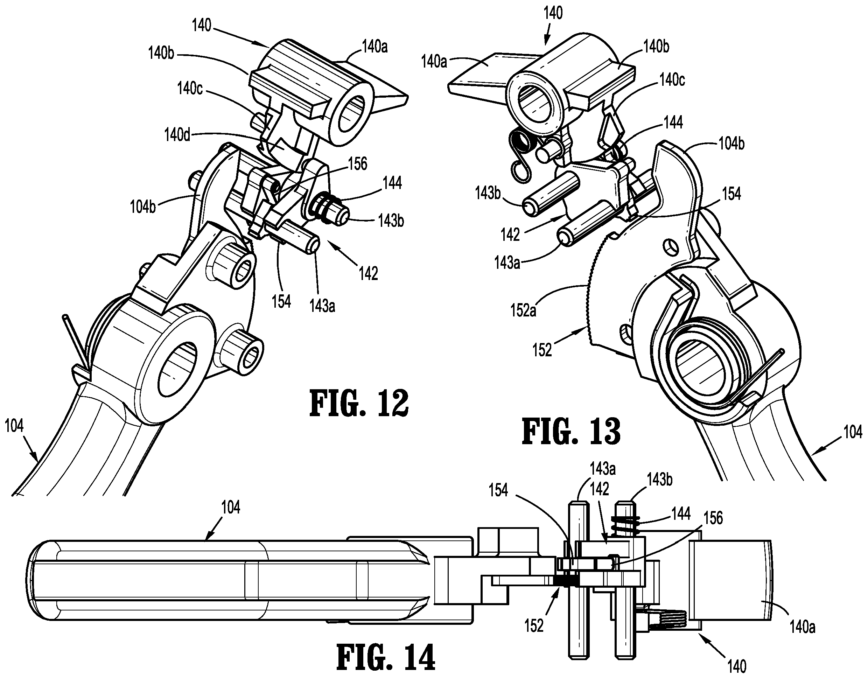

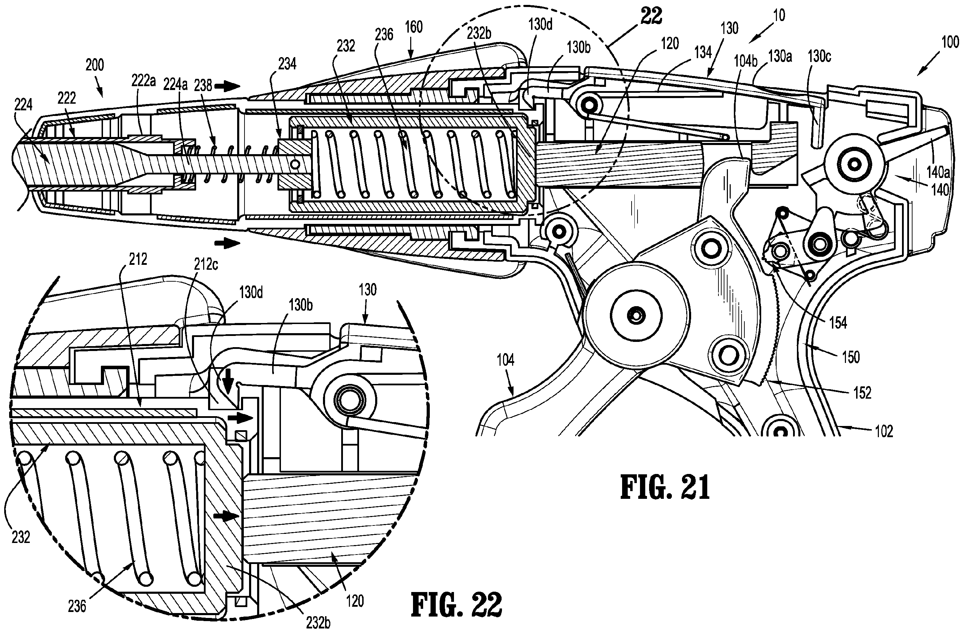

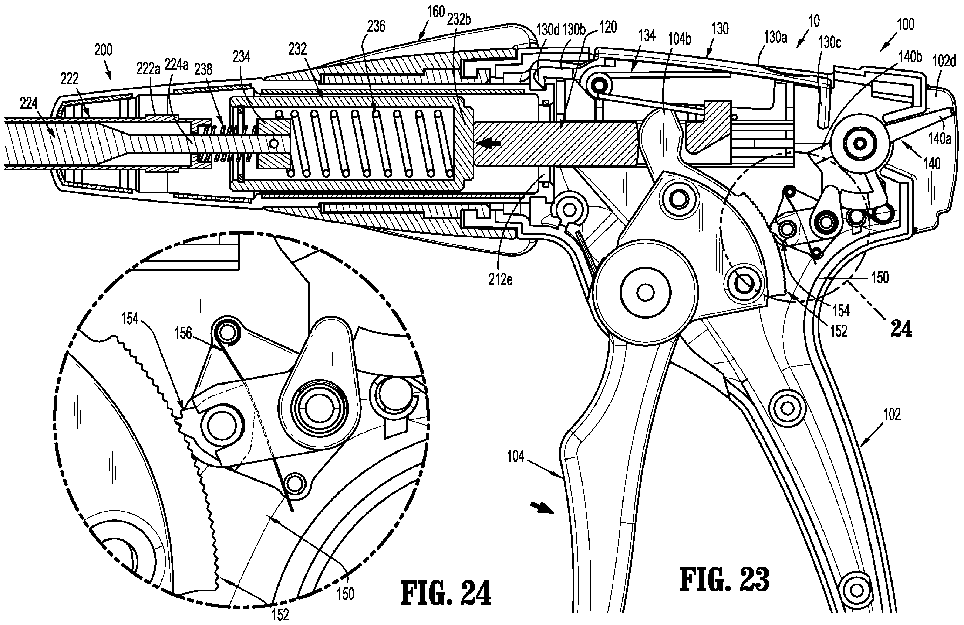

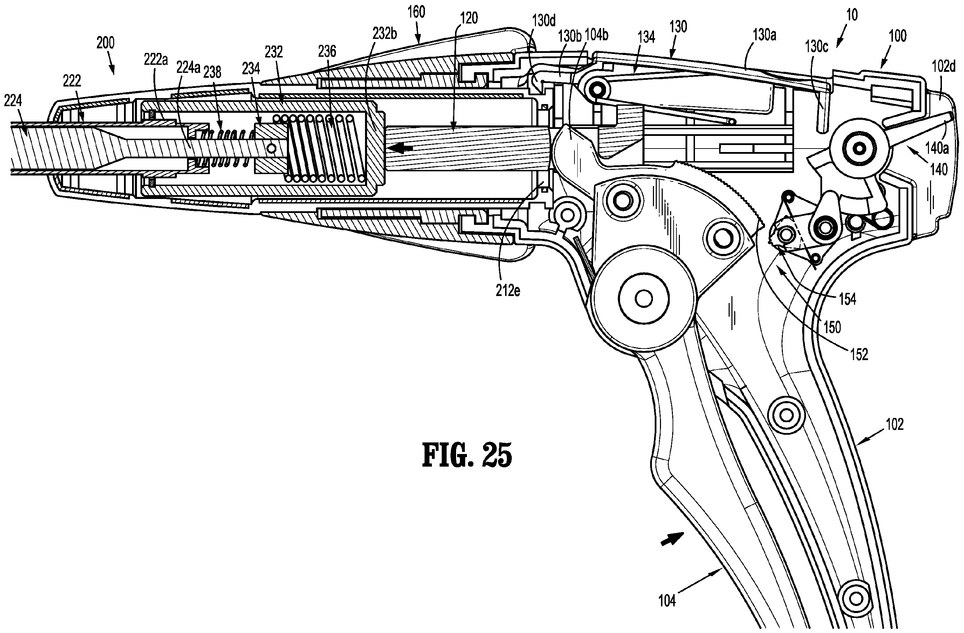

Referring now to FIGS. 1-29, an endoscopic surgical clip applier in accordance with an embodiment of the present disclosure, and assembly in a particular configuration, is generally designated as 10. Surgical clip applier 10 generally includes a reusable handle assembly or actuation assembly 100, at least one disposable or reusable endoscopic assembly 200 selectively connectable to and extendable distally from handle assembly 100; and optionally at least one disposable surgical clip cartridge assembly (not shown) selectively loadable into a shaft assembly of a respective endoscopic assembly 200.

Briefly, the shaft assembly of endoscopic assembly 200 may have various outer diameters such as, for example, about 5mm or about 10mm, depending on intended use. Further, the shaft assembly may have various relatively elongated or shortened lengths depending on intended use, such as, for example, in bariatric surgery. In one embodiment, in bariatric surgery, the shaft assembly may have a length of between about 30cm and about 40cm. Further, the shaft assembly may be configured to fire and form a specific type of surgical clip, either individually or multiply. However one skilled in the art should appreciate that the shaft assembly may have any length in excess of about 30cm and the present disclosure is not limited to any of the above identified lengths.

In accordance with the present disclosure, as will be discussed in greater detail below, an endoscopic assembly or a surgical clip cartridge assembly (not shown) may be loaded with a particularly sized set of surgical clips (e.g., relatively small surgical clips, relatively medium surgical clips, or relatively large surgical clips). It is contemplated that clip cartridge assemblies may be configured to be selectively loaded into the shaft assembly of a respective endoscopic assembly 200, and to be actuated by the same or common handle assembly 100, to fire and form the surgical clip(s) loaded therein onto underlying tissue and/or vessels.

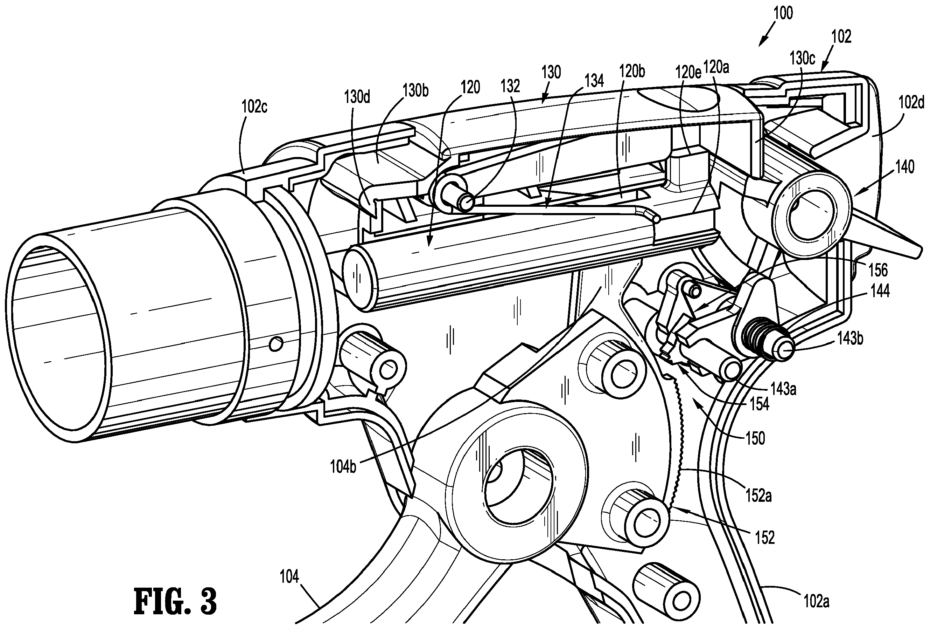

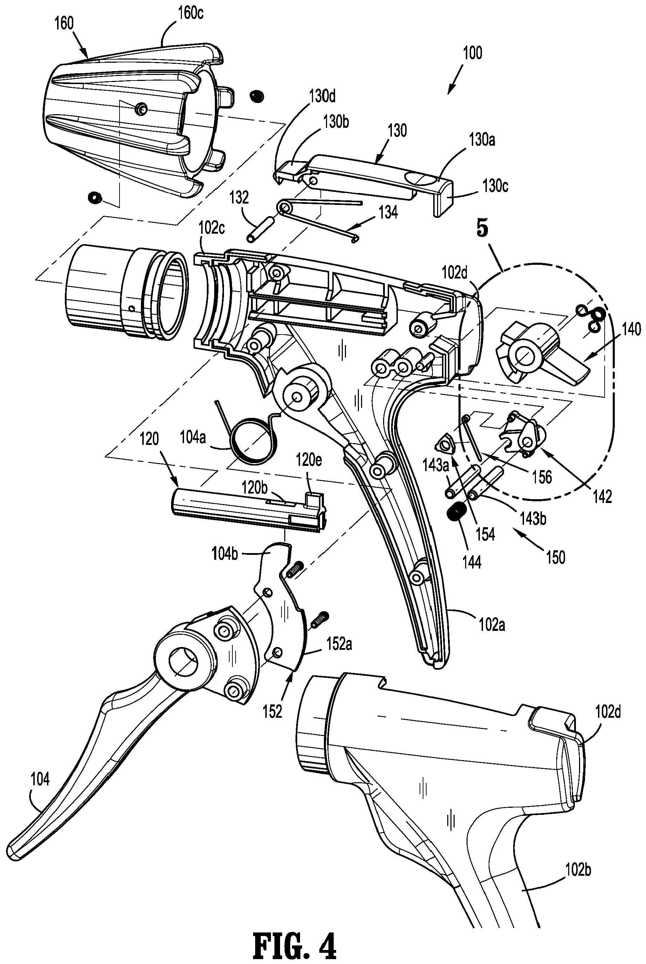

Referring now to FIGS. 1-14, handle assembly 100 of surgical clip applier 10 is shown and will be described. Handle assembly 100 includes a housing 102 having a first or right side half-section 102a and a second or left side half-section 102b. Housing 102 of handle assembly 100 further includes or defines, as seen in FIGS. 3 and 4, a nose 102c. Housing 102 of handle assembly 100 may be formed of a suitable plastic or thermoplastic material. It is further contemplated that housing 102 of handle assembly 100 may be fabricated from stainless steel of the like.

Handle assembly 100 includes a trigger 104 pivotably supported between right side half-section 102a and left side half-section 102b of housing 102. Trigger 104 is biased by a biasing member 104a (e.g., a return spring, compression spring or torsion spring) to an un-actuated condition. Specifically, biasing member 104a (FIG. 4) acts on a feature of trigger 104 and on a feature of housing 102 to bias or urge trigger 104 to the un-actuated condition. Trigger 104 includes a drive arm 104b extending therefrom. Drive arm 104b may be integrally formed therewith or may be separately and fixedly secured to trigger 104. Drive arm 104b may define a curved, radiused or filleted upper distal surface.

As illustrated in FIGS. 3, 4 and 8-14, trigger 104 supports or is provided with at least one linear rack 152 of teeth 152a of a ratchet assembly 150, as will be described in detail below.

With reference to FIGS. 3, 4, 11, handle assembly 100 includes a drive plunger 120 operatively connected to trigger 104. Specifically, drive plunger 120 is slidably supported within housing 102 and defines a pair of opposed, axially extending slots 120a formed in an outer surface thereof. Slots 120a of drive plunger 120 are configured to slidably engage or receive opposed tabs 102d of housing 102. Drive plunger 120 further defines a proximally extending trigger slot 120b formed in a proximal portion thereof for operatively receiving drive arm 104b of trigger 104. Trigger slot 120b defines a distal surface or wall 120c against which a distal surface of drive arm 104b of trigger 104 contacts in order to distally advance drive plunger 120 during an actuation of trigger 104.

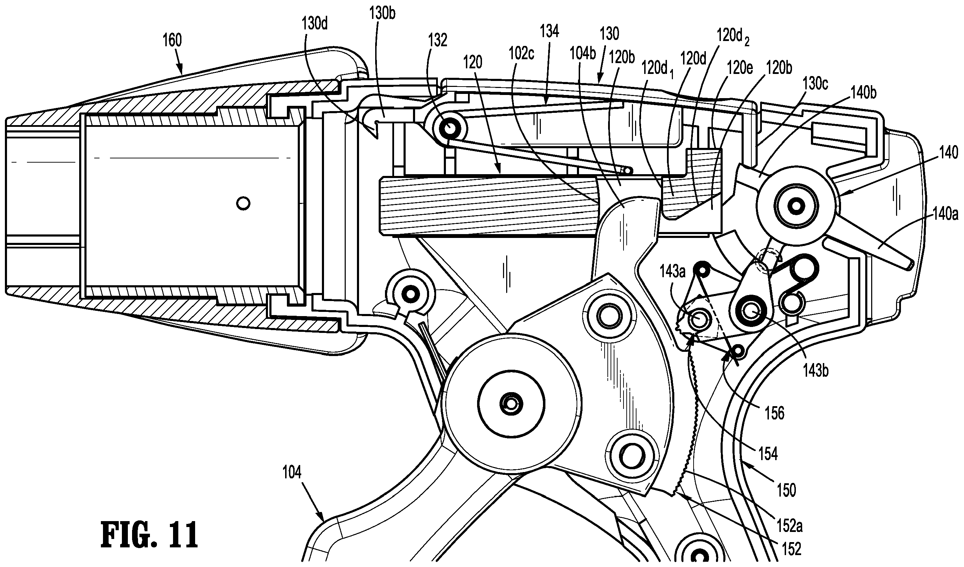

Drive plunger 120 further includes a tooth 120d (FIG. 11) projecting into trigger slot 120b. Tooth 120d projects substantially toward trigger 104 and includes a distal surface or wall 120d1 (spaced proximally from distal surface or wall 120c of drive plunder 120), and a proximal, angled wall 120d2 tapering to a relatively smaller height in a proximal direction.

Drive plunger 120 additionally includes a tab or fin 120e projecting from a surface thereof. Tab 120e of drive plunger 120 may be substantially aligned or in registration with tooth 120d of drive plunger 120. Tab 120e of drive plunger 120 may project in a direction substantially opposite to tooth 120d of drive plunger 120 or to trigger 104.

With reference to FIGS. 1-4 and 11, handle assembly 100 includes an endoscopic assembly release lever 130 pivotally supported on and connected to housing 102 via a pivot pin 132. Pivot pin 132 is supported in housing 102. Release lever 130 includes a proximal end 130a extending proximally of pivot pin 132. Proximal end 130a of release lever 130 includes a wall 130c dimensioned to extend toward a pawl switch 140 of handle assembly 100, as will be described in greater detail below.

Release lever 130 includes a distal end 130b extending distally of pivot pin 132. Distal end 130b of release lever 130 includes a catch or tooth 130d projecting therefrom, in a direction towards drive plunger 120. Catch 130d may be located distal of drive plunger 120.

A biasing member 134, in the form of a leaf spring, may be provided which tends to bias distal end 130b and catch 130d of release lever 130 towards drive plunger 120 of handle assembly 100, and tends to bias proximal end 130a of release lever 130 away from pawl switch 140. Specifically, biasing member 134 tends to maintain catch 130d of release lever 130 in engagement with an engagement feature (e.g., annular channel 212c) of endoscopic assembly 200, as will be described in greater detail below.

With reference to FIGS. 3, 4 and 11-14, as mentioned above, handle assembly 100 includes a ratchet assembly 150 supported within housing 102. Ratchet assembly 150 includes, as also mentioned above, at least one linear rack 152 of teeth 152a supported on and projecting from trigger 104. Ratchet assembly 150 further includes a ratchet pawl 154 pivotally connected to housing 102 by a pawl pin at a location wherein pawl 154 is in substantial operative engagement with rack 152. Ratchet assembly 150 further includes a pawl spring 156 configured and positioned to bias pawl 154 into operative engagement with rack 152. Pawl spring 156 functions to maintain the tooth or teeth 154a of pawl 154 in engagement with teeth 152a of rack 152, as well as to maintain pawl 154 in a rotated or canted position.

Pawl 154 is engagable with rack 152 to restrict longitudinal movement of rack 152 and, in turn, trigger 104. In use, as trigger 104 is actuated (from a fully un-actuated position), rack 152 is also moved, into engagement with pawl 154. Rack 152 has a length which allows pawl 154 to reverse and advance back over rack 152, when rack 152 changes between proximal or distal movement, as trigger 104 reaches a fully actuated or fully un-actuated position. The relative lengths and sizes of rack 152 of ratchet assembly 150, trigger 104 and drive plunger 120 define a stroke length of trigger 104, drive plunger 120 or handle assembly 100 (e.g., a "full stroke").

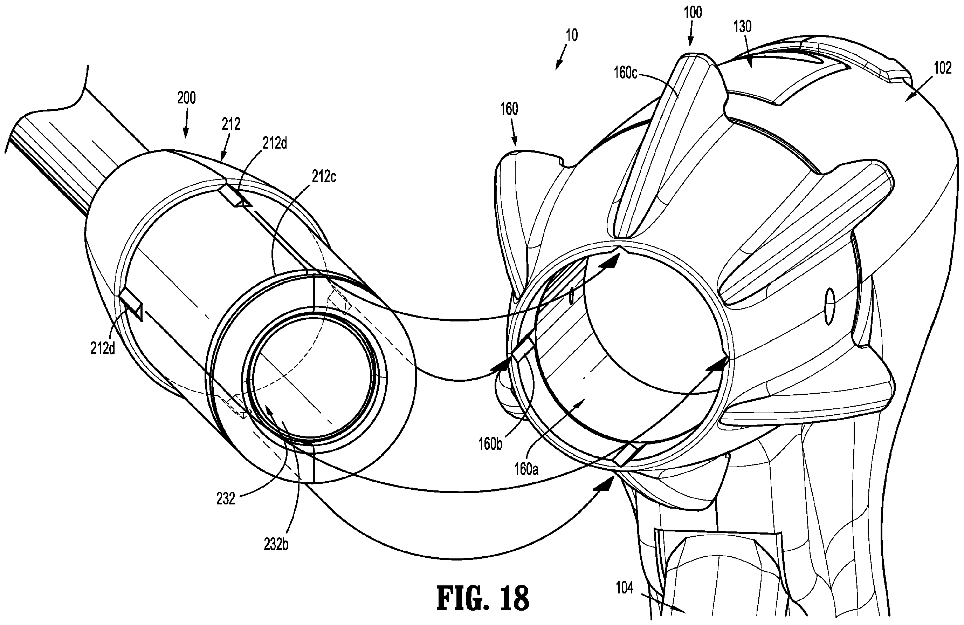

Turning now to FIGS. 1, 2, 4, 11 and 18, handle assembly 100 includes a rotation knob 160 rotatably supported on nose 102c of housing 102. Rotation knob 160 includes a central axial bore 160a having an annular array of longitudinally extending grooves 160b (FIG. 18) formed in a surface thereof. Grooves 160b of rotation knob 160 function as clocking and alignment features for the connection of endoscopic assembly 200 with handle assembly 100. Rotation knob 160 further includes a plurality of finger grip ribs 160c projecting from an outer surface thereof.

With reference to FIGS. 3 and 4-14, handle assembly 100 further includes a pawl switch 140 and a pawl actuator 142 each pivotally supported in housing 102. Pawl switch 140 is operatively connected to pawl actuator 142 and is operable to selectively move pawl actuator 142 into or out of engagement with pawl spring 156, and in turn pawl 154, of ratchet assembly 150 whereby pawl 154 may be selectively engaged by pawl spring 156. In this manner, when pawl 154 is moved out of engagement with pawl spring 156, trigger 104 is free to open and close as needed due to pawl 154 having minimal blocking effect on rack 152 of ratchet assembly 150. As such, trigger 104 may be partially actuated (without having to be fully actuated), and may be returnable to a fully un-actuated position. Such a feature permits the user to partially squeeze or actuate trigger 104 for performing a cholangiogram procedure or the like.

Pawl switch 140 includes a finger lever 140a projecting from housing 102, whereby pawl switch 140 may be actuated by a finger of a user. Housing 102 of handle assembly 100 may be provided with guard walls 102d disposed on opposed sides of finger lever 140a in order to inhibit inadvertent actuation of pawl switch 140. Pawl switch 140 is movable, upon actuation of finger lever 140a, between a first position in which ratchet assembly 150 is "on" or "activated", and a second position in which ratchet assembly 150 is "off" or "deactivated." It is contemplated that pawl switch 140, and in turn ratchet assembly 150, default to the first position.

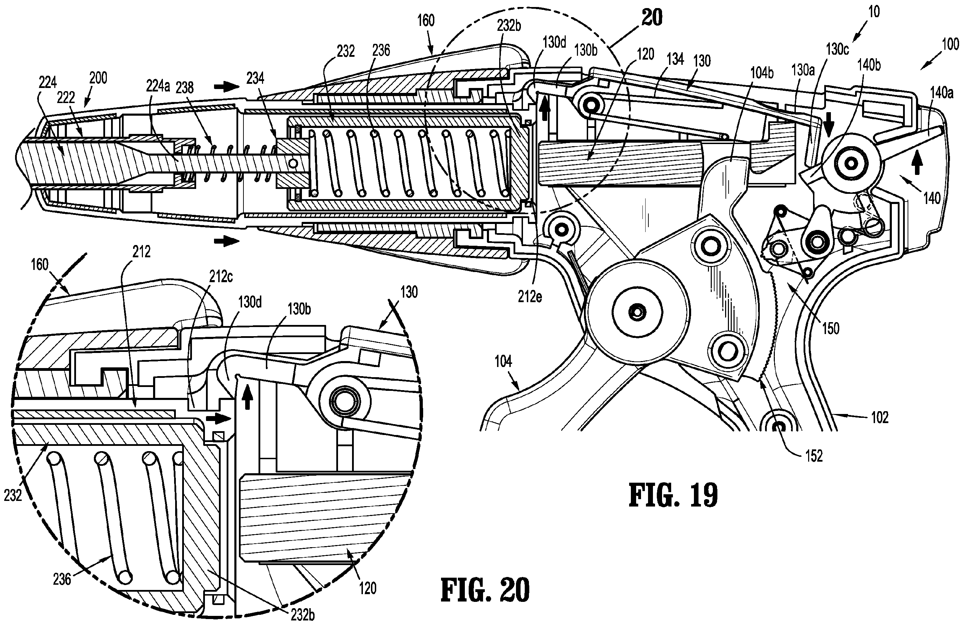

Pawl switch 140 further includes a first flange 140b projecting a first distance from a pivot point thereof, and a second flange 140c projecting a second distance from the pivot point thereof, wherein the projection of the second flange 140c is greater than the projection of the first flange 140b. First flange 140b of pawl switch 140 is selectively engagable by wall 130c of proximal end 130a of release lever 130. In this manner, each time an endoscopic assembly 200 is attached to handle assembly 100, and release lever 130 is actuated, wall 130c of release lever 130 engages first flange 140b of pawl switch 140 to move pawl switch to the first position (FIGS. 19-22).

Pawl switch 140 also includes a ramp or camming surface 140d projecting therefrom which selectively engages a tab or finger 142a of pawl actuator 142 to slidably move pawl actuator 142, and in turn pawl spring 156, into and out of operative engagement/registration with/from pawl 154.

Pawl actuator 142 is pivotally connected to housing 102 and operatively connected to pawl switch 140 such that actuation of pawl switch 140 actuates pawl actuator 142. Pawl actuator 142 is slidably supported on a pair of support pins 143a, 143b, and a biasing member 144 is provided to bias pawl actuator 142 against pawl switch 140. In operation, with reference to FIGS. 11-14, when pawl switch 140 is actuated to the second position, ramp or camming surface 140d of pawl switch 140 acts on tab 142a of pawl actuator 142 to transversely slide pawl actuator 142 along support pins 143a, 143b and move pawl spring 156 out of operative engagement/registration with pawl 154, thereby disabling the operability of ratchet assembly 150. Also, as pawl actuator 142 is slid transversely along support pins 143a, 143b, pawl actuator 142 biases biasing member 144.

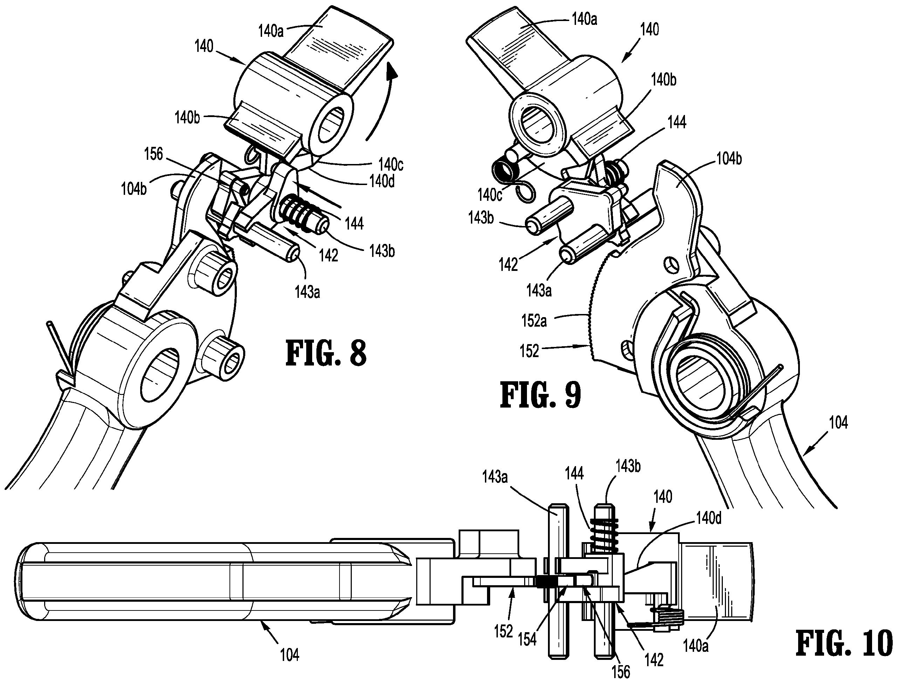

Further in operation, with reference to FIGS. 8-10, when pawl switch 140 is actuated to the first position, ramp or camming surface 140d of pawl switch 140 is moved to permit biasing member 144 to expand and transversely slide pawl actuator 142 along support pins 143a, 143b, whereby pawl spring 156 is moved back into operative engagement/registration with pawl 154, thereby enabling or re-enabling the operability of ratchet assembly 150.

Turning now to FIGS. 1, 2, 16 and 17, an embodiment of an endoscopic assembly 200, of surgical clip applier 10, is shown and described. Endoscopic assembly 200 includes a hub assembly 210, a shaft assembly 220 extending from hub assembly 210, and a pair of jaws 250 pivotally connected to a distal end of shaft assembly 220. It is contemplated that endoscopic assembly 200 may be configured to close, fire or form surgical clips similar to those shown and described in U.S. Patent No. 4,834,096, the entire content of which is incorporated herein by reference.

Hub assembly 210 functions as an adapter assembly which is configured for selective connection to rotation knob 160 and nose 102c of housing 102 of handle assembly 100. Hub assembly 210 includes an outer housing 212 having a cylindrical outer profile. Outer housing 212 includes a first or right side half section 212a, and a second or left side half section 212b. Outer housing 212 of hub assembly 210 defines an outer annular channel 212c formed in an outer surface thereof, and at least one (or an annular array) of axially extending ribs 212d projecting from an outer surface thereof. Outer annular channel 212c of outer housing 212 of endoscopic assembly 200 is configured to receive catch 130d of release lever 130 of handle assembly 100 (FIGS. 19-22) when endoscopic assembly 200 is coupled to handle assembly 100.

Ribs 212d of outer housing 212 function as a clocking/alignment feature during connection of endoscopic assembly 200 and handle assembly 100 with one another, wherein ribs 212d of outer housing 212 of endoscopic assembly 200 are radially and axially aligned with respective grooves 160b of rotation knob 160 of handle assembly 100. During connection of endoscopic assembly 200 and handle assembly 100, ribs 212d of outer housing 212 of endoscopic assembly 200 are slidably received in respective grooves 160b of rotation knob 160 of handle assembly 100.

The connection of hub assembly 210 of endoscopic assembly 200 with rotation knob 160 of handle assembly 100 enables endoscopic assembly 200 to rotate 360°, about a longitudinal axis thereof, relative to handle assembly 100.

Outer housing 212 of hub assembly 210 further defines an open proximal end 212e configured to slidably receive a distal end of drive plunger 120 of handle assembly 100, when endoscopic assembly 200 is coupled to handle assembly 100 and/or when surgical clip applier 10 is fired.

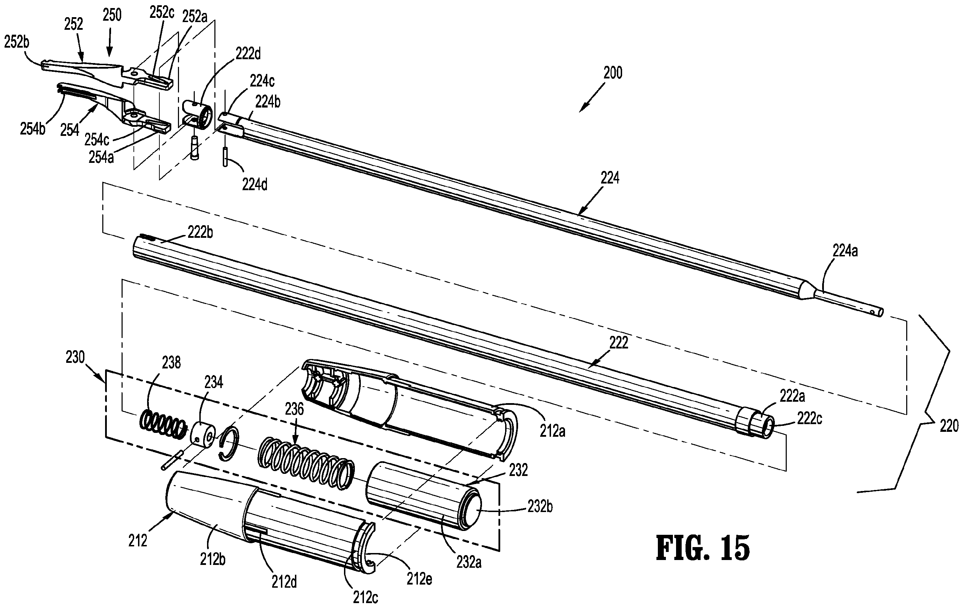

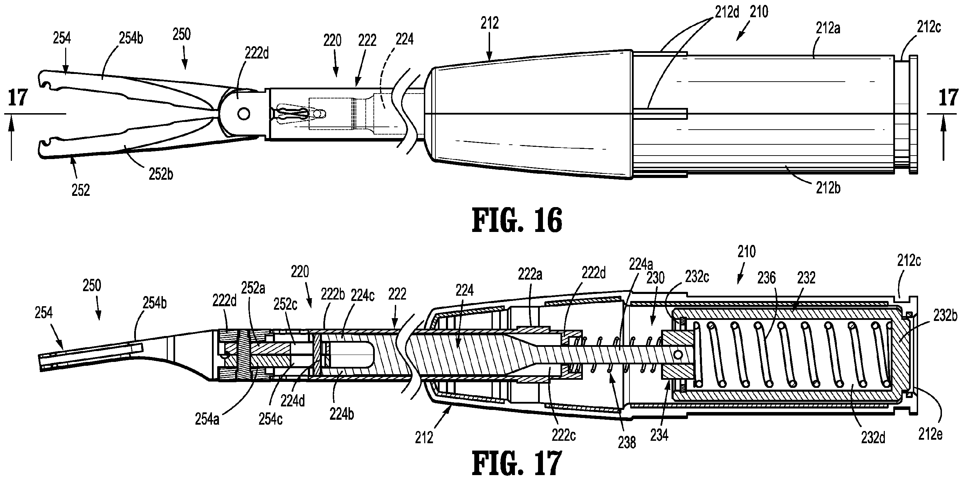

As mentioned above, endoscopic assembly 200 includes a shaft assembly 220 extending distally from hub assembly 210. Shaft assembly 220 includes an elongate outer tube 222 having a proximal end 222a supported and secured to outer housing 212 of hub assembly 210, a distal end 222b projecting from outer housing 212 of hub assembly 210, and a lumen 222c (FIGS. 15 and 17) extending longitudinally therethrough. Distal end 222b of outer tube 222 supports or defines an outer clevis 222d for pivotally supporting a pair of jaws 250, as will be described in greater detail below.

Shaft assembly 220 further includes an inner shaft 224 slidably supported within lumen 222c of outer tube 222. Inner shaft 224 includes a proximal end 224a projecting proximally from proximal end 222a of outer tube 222, and a distal end 224b defining an inner clevis 224c for supporting a cam pin 224d which engages camming slots 252c, 254c of a pair of jaws 250, as will be described in greater detail below.

With reference to FIGS. 15 and 17, hub assembly 210 includes a drive assembly 230 supported within outer housing 212 thereof. Drive assembly 230 includes a cartridge cylinder 232 having a cup-like configuration, wherein cartridge cylinder 232 includes an annular wall 232a, a proximal wall 232b supported at and closing off a proximal end of annular wall 232a, an open distal end 232c, and a cavity or bore 232d defined therewithin.

Drive assembly 230 also includes a cartridge plunger 234 slidably supported within bore 232d of cartridge cylinder 232. Cartridge plunger 234 is fixedly supported on inner shaft 224, at the proximal end 224a thereof. Cartridge plunger 234 is sized and configured for slidable receipt within bore 232d of cartridge cylinder 232 of drive assembly 230. A ring, flange or the like 235 may be fixedly supported at a distal end of bore 232d of cartridge cylinder 232, through which proximal end 224a of cartridge plunger 234 extends and which functions to maintain cartridge plunger 234 within bore 232d of cartridge cylinder 232.

Drive assembly 230 includes a first biasing member 236 (e.g., a compression spring) disposed within bore 232d of cartridge cylinder 232. Specifically, first biasing member 236 is interposed between proximal wall 232b of cartridge cylinder 232 and a proximal surface of cartridge plunger 234. First biasing member 236 has a first spring constant "K1" which is relatively more firm or more stiff, as compared to a second spring constant "K2" of a second biasing member 238, as is described in detail below.

Drive assembly 230 further includes a second biasing member 238 (e.g., a compression spring) supported on proximal end 224a of inner shaft 224. Specifically, second biasing member 238 is interposed between a proximal flange 222d of outer tube 222 and a distal surface of cartridge plunger 234. Second biasing member 238 has a second spring constant "K2" which is relatively less firm or less stiff, as compared to the first spring constant "K1" of first biasing member 236.

As illustrated in FIGS. 15 and 17, endoscopic assembly 200 includes a pair of jaws 250 pivotally supported in a clevis 222d at distal end 222b of outer tube 222 by a pivot pin 256. The pair of jaws 250 includes a first jaw 252 and a second jaw 254. Each jaw 252, 254 includes a respective proximal end 252a, 254a, and a respective distal end 252b, 254b, wherein proximal ends 252a, 254a and distal ends 252b, 254b of jaws 252, 254 are pivotable about pivot pin 256. Each proximal end 252a, 254a of respective jaws 252, 254 defines a cam slot 252c, 254c therein which is sized and configured to receive cam pin 224d of inner shaft 224. In use, as inner shaft 224 is axially displaced relative to outer shaft 222, inner shaft 224 translated cam pin 224d thereof through cam slot 252c, 254c of jaws 252, 254 to thereby open or close the pair of jaws 250.

When the pair of jaws 250 are in an open position, and a new, unformed or open surgical clip (not shown) is located or loaded within the distal ends 252b, 254b of jaws 252, 254 of the pair of jaws 250, as inner shaft 224 is moved distally relative to outer shaft 222, cam pin 224d is translated through cam slots 252c, 254c of jaws 252, 254. As cam pin 224d is translated through cam slots 252c, 254c of jaws 252, 254 the distal ends 252b, 254b of jaws 252, 254 are moved to the closed or approximated position to close and/or form the surgical clip located or loaded therewithin.

The dimensions of jaws 252, 254 and of cam slots 252c, 254c of jaws 252, 254 determines an overall length required to move jaws 252, 254 from a fully open position to a fully closed position, defining a closure stroke length of the pair of jaws 250.

With reference now to FIGS. 19-25, an operation or firing of surgical clip applier 10, including endoscopic assembly 200 operatively connected to handle assembly 100, is shown and described. With endoscopic assembly 200 operatively connected to handle assembly 100, and with a new, unformed or open surgical clip (not shown) is located or loaded within the distal ends 252b, 254b of jaws 252, 254 of the pair of jaws 250, as trigger 104 of handle assembly 100 is actuated drive bar 104b of trigger 104 acts on drive plunger 120 to distally advance drive plunger 120. As trigger 104 is actuated, pawl 154 of ratchet assembly 150 begins to engage rack 152 thereof. With pawl 154 engaged with rack 152, trigger 104 may not return to a fully unactuated position until trigger 104 completes a full actuation or stroke thereof.

As drive plunger 120 is distally advanced, a distal end of drive plunger 120 presses against proximal wall 232b of cartridge cylinder 232 of drive assembly 230 of endoscopic assembly 200 to distally advance cartridge cylinder 232. Due to first spring constant "K1" of first biasing member 236 being larger or greater than second spring constant "K2" of second biasing member 238, as cartridge cylinder 232 is advanced distally, cartridge cylinder 232 distally advances first biasing member 236, which in turn acts on cartridge plunger 234 to distally advance cartridge plunger 234. As cartridge plunger 234 is distally advanced, cartridge plunger 234 distally advances inner shaft 224 relative to outer shaft 222. Being that second biasing member 238 is interposed between proximal flange 222d of outer tube 222 and distal surface of cartridge plunger 234, as cartridge plunger 234 is distally advanced, cartridge plunger 234 also compresses second biasing member 238.

As inner shaft 224 is distally advanced relative to outer shaft 222, inner shaft 224 distally advances cam pin 224d through cam slot 252c, 254c of jaws 252, 254 to close the pair of jaws 250 and to close and/or form the surgical clip (not shown) loaded within the pair of jaws 250. Cam pin 224d of inner shaft 224 is advanced distally until cam pin 224d reaches an end of cam slots 252c, 254c of jaws 252, 254 of the pair of jaws 250 and/or until the distal ends 252b, 254b of jaws 252, 254 of the pair of jaws 250 are fully approximated against one another (e.g., in contact with one another or fully closed on the surgical clip (not shown)), whereby cam pin 224d may not have reached the end of cam slots 252c, 254c of jaws 252, 254. This position may be considered a hard stop of the pair of jaws 250. The axial distance that cam pin 224d has traveled from a proximal-most position thereof to when cam pin 224d reaches the end of cam slots 252c, 254c of jaws 252, 254 or when the distal ends 252b, 254b of jaws 252, 254 of the pair of jaws 250 are fully approximated against one another, may also define the closure stroke length of the pair of jaw 250.

When the pair of jaws 250 have reached the hard stop, or when the cam pin 224d has reached an end of the closure stroke length, pawl 154 of ratchet assembly 150 of handle assembly 100 may not have cleared rack 152 thereof, and thus blocks or prevents trigger 104 from returning to a fully unactuated position thereof. Since the pair of jaws 250 cannot close any further, and since cam pin 224d cannot be advanced distally any further, inner shaft 222 is also stopped from further distal advancement. However, as mentioned above, in order to return trigger 104 to the fully unactuated position, trigger 104 must first complete the full actuation stroke thereof. As such, as trigger 104 is further actuated to complete the full stroke thereof, as drive plunger 120 is continued to be driven distally, the distal end of drive plunger 120 continues to press against proximal wall 232b of cartridge cylinder 232 of drive assembly 230 of endoscopic assembly 200 to continue to distally advance cartridge cylinder 232.

With inner shaft 222, and in turn cartridge plunger 234, stopped from any further distal advancement, as cartridge cylinder 232 is continued to be advanced distally, cartridge cylinder 232 begins to and continues to compress first biasing member 236 until such time that pawl 154 of ratchet assembly 150 of handle assembly 100 clears and disengages rack 152 thereof. With pawl 154 of ratchet assembly 150 clear and disengaged from rack 152, trigger 104 may be released and returned to the fully unactuated position by hand, by a return spring 104a of trigger 104 and/or by first biasing member 236 and second biasing member 238 of endoscopic assembly 200.

In accordance with the present disclosure, the trigger stroke length for trigger 104 of handle assembly 100 is constant or fixed, while the closure stroke length of the pair of jaws 250 may vary depending on the particular endoscopic assembly 200 connected to handle assembly 100. For example, particular endoscopic assemblies 200 may require the pair of jaws 250 thereof to travel a relatively greater or smaller distance in order to complete a full opening and closing thereof. As such, various sized and dimensioned endoscopic assemblies, including a hub assembly in accordance with the present disclosure, substantially similar to hub assembly 210, may be connected to the universal handle assembly 100 and be actuatable by the universal handle assembly 100.

Accordingly, various endoscopic assemblies, constructed in accordance with the principles of the present disclosure, may be provided which are also capable of firing or forming or closing surgical clips of various sizes, materials, and configurations, across multiple platforms for multiple different manufactures.

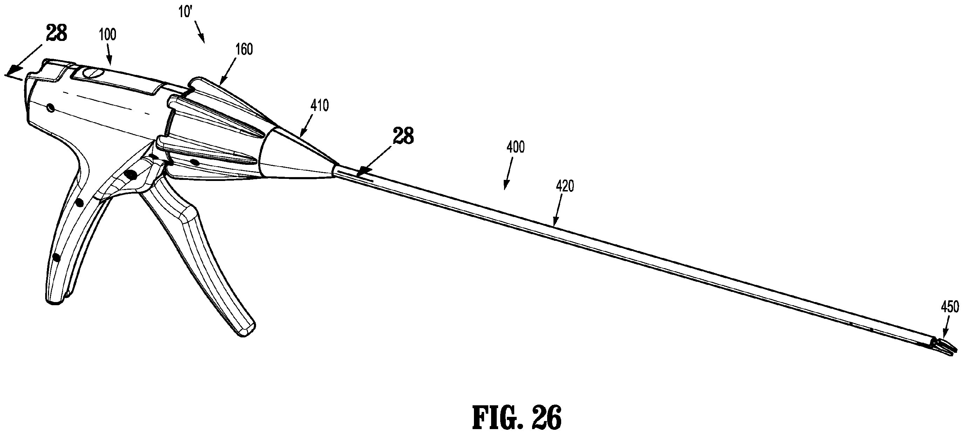

Turning now to FIGS. 26-29, an endoscopic surgical clip applier, in accordance with the present disclosure, and assembly in another configuration, is generally designated as 10'. Surgical clip applier 10' generally includes reusable handle assembly 100, at least one disposable or reusable endoscopic assembly 400 selectively connectable to and extendable distally from handle assembly 100; and optionally at least one disposable surgical clip cartridge assembly (not shown) selectively loadable into a shaft assembly of a respective endoscopic assembly 400.

Turning now to FIGS. 1, 2, 16 and 17, an embodiment of an endoscopic assembly 400, of surgical clip applier 10', is shown and described. Endoscopic assembly 400 includes a hub assembly 410, a shaft assembly 420 extending from hub assembly 410, and a pair of jaws 450 pivotally connected to a distal end of shaft assembly 420. It is contemplated that endoscopic assembly 400 may be configured to close, fire or form surgical clips similar to those shown and described in U.S. Patent Nos. 7,819,886 or 7,905,890, the entire contents of each of which is incorporated herein by reference.

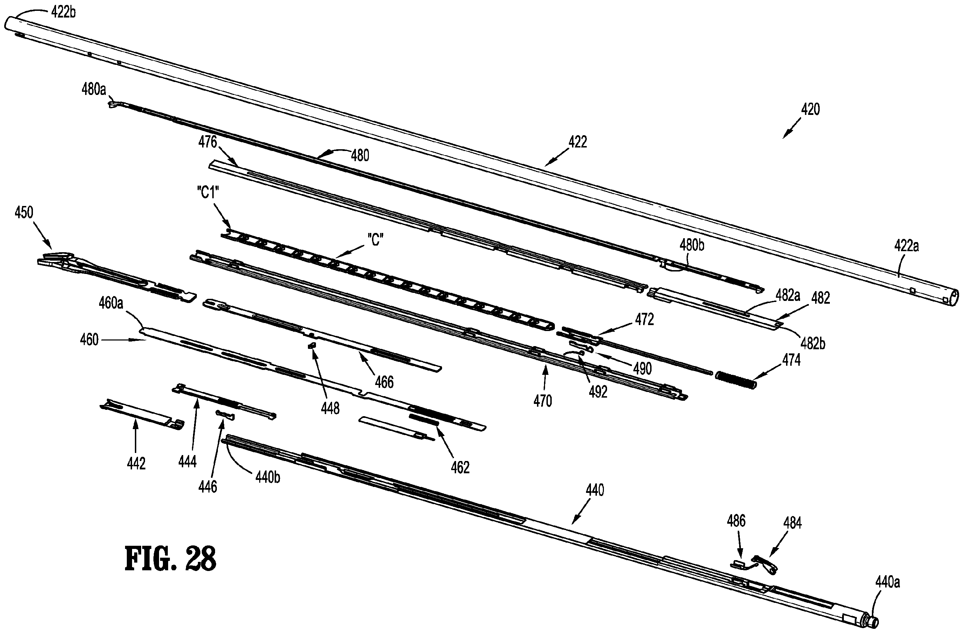

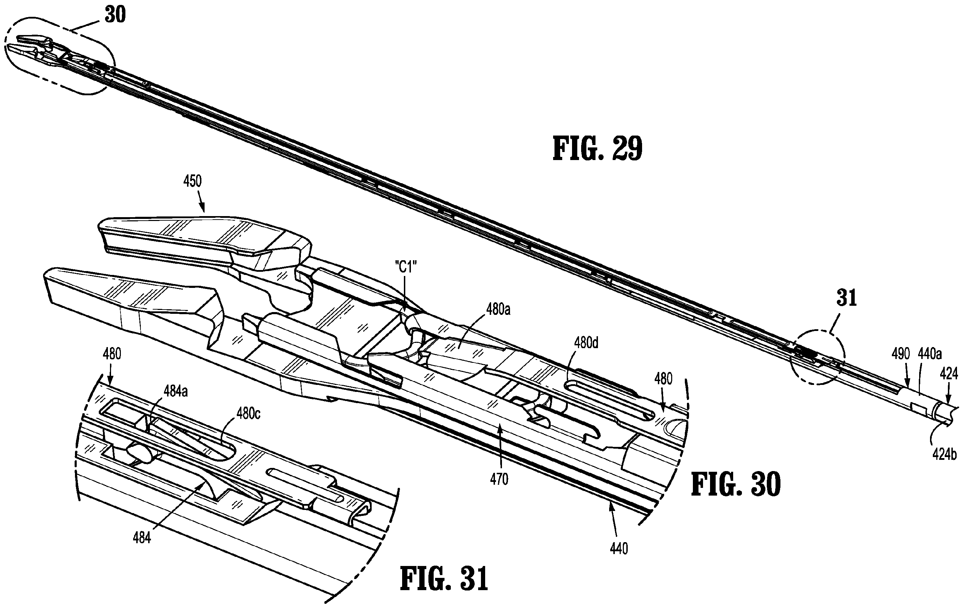

Hub assembly 410 also functions as an adapter assembly which is configured for selective connection to rotation knob 160 and nose 102c of housing 102 of handle assembly 100. Hub assembly 410 includes an outer housing 412 having a cylindrical outer profile. Outer housing 412 includes a first or right side half section 412a, and a second or left side half section 412b. Outer housing 412 of hub assembly 410 defines an outer annular channel 412c formed in an outer surface thereof, and at least one (or an annular array) of axially extending ribs 412d projecting from an outer surface thereof. Outer annular channel 412c of outer housing 412 of endoscopic assembly 400 is configured to receive catch 130d of release lever 130 of handle assembly 100 (FIGS. 28 and 29) when endoscopic assembly 400 is coupled to handle assembly 100.

Ribs 412d of outer housing 412 function as a clocking/alignment feature during connection of endoscopic assembly 400 and handle assembly 100 with one another, wherein ribs 412d of outer housing 412 of endoscopic assembly 400 are radially and axially aligned with respective grooves 160b of rotation knob 160 (FIG. 18) of handle assembly 100. During connection of endoscopic assembly 400 and handle assembly 100, ribs 412d of outer housing 412 of endoscopic assembly 400 are slidably received in respective grooves 160b of rotation knob 160 of handle assembly 100.

The connection of hub assembly 410 of endoscopic assembly 400 with rotation knob 160 of handle assembly 100 enables endoscopic assembly 400 to rotate 360°, about a longitudinal axis thereof, relative to handle assembly 100.

Outer housing 412 of hub assembly 410 further defines an open proximal end 412e configured to slidably receive a distal end of drive plunger 120 of handle assembly 100, when endoscopic assembly 400 is coupled to handle assembly 100 and/or when surgical clip applier 10' is fired.

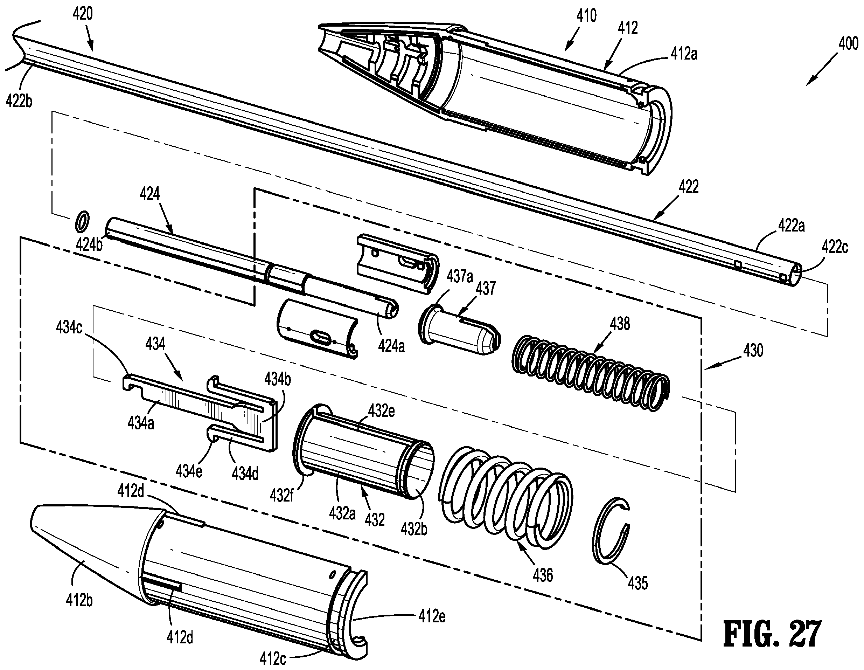

As mentioned above, endoscopic assembly 400 includes a shaft assembly 420 extending distally from hub assembly 410. Shaft assembly 420 includes an elongate outer tube 422 having a proximal end 422a supported and secured to outer housing 412 of hub assembly 410, a distal end 422b projecting from outer housing 412 of hub assembly 410, and a lumen 422c (FIG. 27) extending longitudinally therethrough. Distal end 422b of outer tube 422 supports a pair of jaws 450.

Shaft assembly 420 further includes an inner shaft 424 slidably supported within lumen 422c of outer tube 422. Inner shaft 424 includes a proximal end 424a projecting proximally from proximal end 422a of outer tube 422, and a distal end 424b configured to actuate the pair of jaws 450 to form a surgical clip (not shown) that has been loaded into the pair of jaws 450. Proximal end 424a, as illustrated in FIGS. 28 and 29, may define a hook 424c or other translational force coupling feature.

With reference to FIGS. 27-29, hub assembly 410 includes a drive assembly 430 supported within outer housing 412 thereof. Drive assembly 430 includes a cartridge cylinder 432 having a cup-like configuration, wherein cartridge cylinder 432 includes a longitudinally split annular wall 432a, a proximal wall 432b supported at and closing off a proximal end of annular wall 432a, an open distal end 432c, a cavity or bore 432d defined therewithin, and a pair of axially extending slits 432e. Cartridge cylinder 432 includes an annular flange 432f provided at distal end 432c thereof. A ring, flange or the like 435 may be fixedly supported at a proximal end of cartridge cylinder 432.

Drive assembly 430 also includes a cartridge plunger or key 434 slidably supported within bore 432d and within slits 432e of cartridge cylinder 432. Cartridge plunger 434 is selectively connectable to proximal end 424a of inner shaft 424. Cartridge plunger 434 is sized and configured for slidable receipt within slits 432e and bore 432d of cartridge cylinder 432 of drive assembly 430. Cartridge plunger 434 includes an elongate stem or body portion 434a having a proximal end 434b, and a distal end 434c, wherein distal end 434c of cartridge plunger 434 is configured for selective connection to proximal end 424a of inner shaft 424. Cartridge plunger 434 further includes a pair of opposed arms 434d supported at the proximal end 434b thereof and which extend in a distal direction along stem 434a and towards distal end 434c. Each arm 434d terminates in a radially extending finger 434e, wherein fingers 434e project from cartridge cylinder 432 when cartridge plunger 434 is disposed within cartridge cylinder 432.

Drive assembly 430 may also include a collar 437 defining a lumen therethrough and through with inner shaft 424 and stem 434a of cartridge plunger 434 extend. Collar 437 includes an outer annular flange 437a extending therefrom.

Drive assembly 430 includes a first biasing member 436 (e.g., a compression spring) disposed about cartridge cylinder 432. Specifically, first biasing member 436 is interposed between ring 435 supported on cartridge cylinder 432 and fingers 434e of cartridge plunger 434. First biasing member 436 has a first spring constant "K1" which is relatively more firm or more stiff, as compared to a second spring constant "K2" of a second biasing member 438, as is described in detail below.

Drive assembly 430 further includes a second biasing member 438 (e.g., a compression spring) supported on stem 434a of cartridge plunger 434 and on collar 437. Specifically, second biasing member 438 is interposed between a flange 437a of collar 437 and proximal end 434b of cartridge plunger 434. Second biasing member 438 has a second spring constant "K2" which is relatively less firm or less stiff, as compared to the first spring constant "K1" of first biasing member 436.

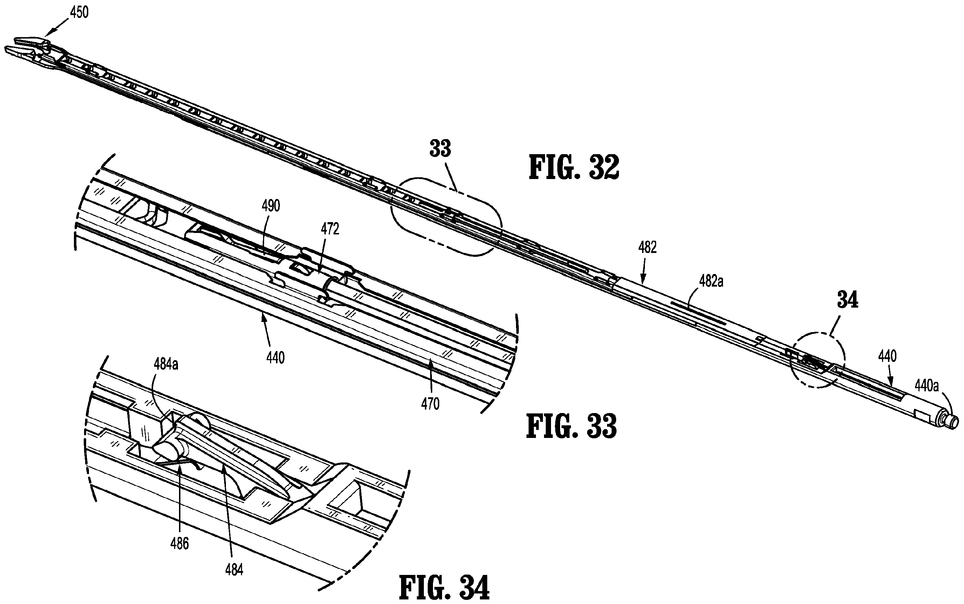

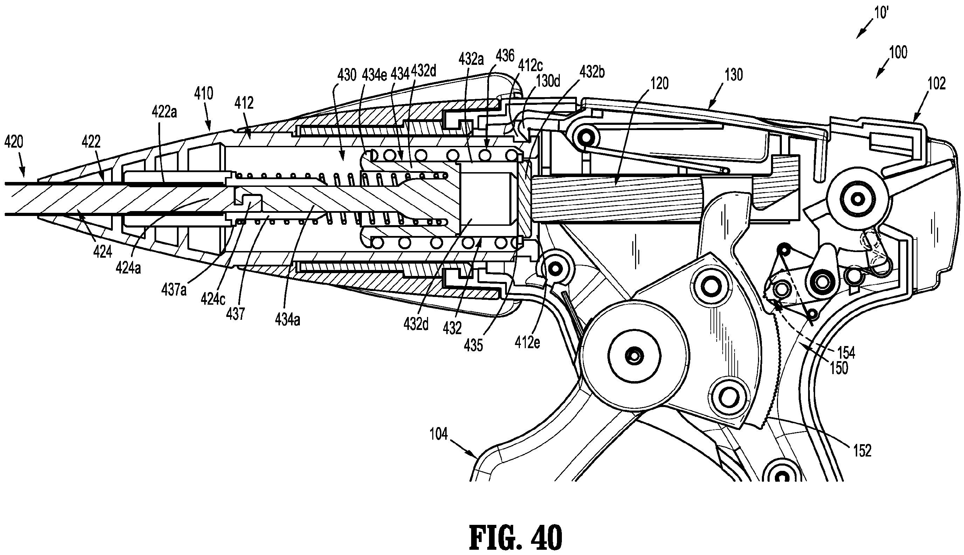

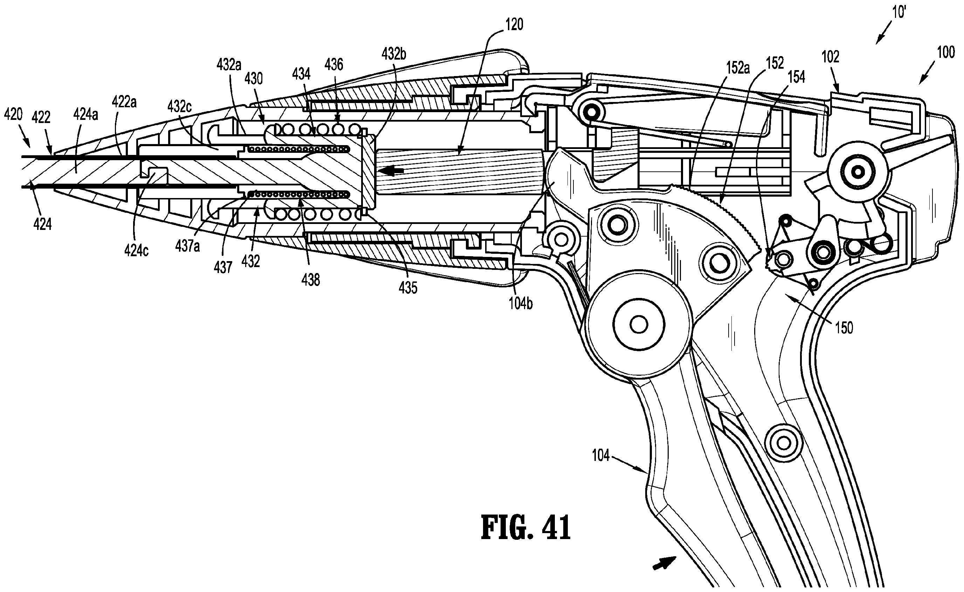

Turning now to FIGS. 26-41, shaft assembly 420 of endoscopic assembly 400 includes at least a spindle 440 slidably supported in lumen 422c of outer tube 422, a wedge plate 460 slidably supported within lumen 422c of outer tube 422 and interposed between the pair of jaws 450 and spindle 440; a clip channel 470 fixedly supported in lumen 422c of outer tube 422 and disposed adjacent the pair of jaws 450 (supported in and extending from distal end 422b of outer tube 422) on a side opposite wedge plate 460, and a pusher bar 480 slidably supported in lumen 422c of outer tube 422 and being disposed adjacent clip channel 470.

Spindle 440 includes a proximal end 440 defining an engagement feature (e.g., a nub or enlarged head) configured to engage a complementary engagement feature provided in distal end 424b of inner shaft 424. Spindle 440 further includes a distal end 440b operatively connected to a jaw cam closure wedge 442 via a slider joint 444. Jaw cam closure wedge 442 is selectively actuatable by spindle 440 to engage camming features of the pair of jaws 450 to close the pair of jaws 450 and form a surgical clip "C" loaded therewithin.

Slider joint 444 supports a latch member 446 for selective engagement with spindle 440. Latch member 446 may be cammed in a direction toward spindle 440, wherein latch member 446 extends into a corresponding slot formed in spindle 440 during actuation or translation of spindle 440. In operation, during distal actuation spindle 400, at a predetermined distance, latch member 446 is mechanically forced or cammed into and engage a channel of spindle 440. This engagement of latch member 446 in the channel of spindle 440 allows slider joint 444 to move together with jaw cam closure wedge 442. Jaw cam closure wedge 442 thus can engage the relevant surfaces of the pair of jaws 450 to close the pair of jaws 450.

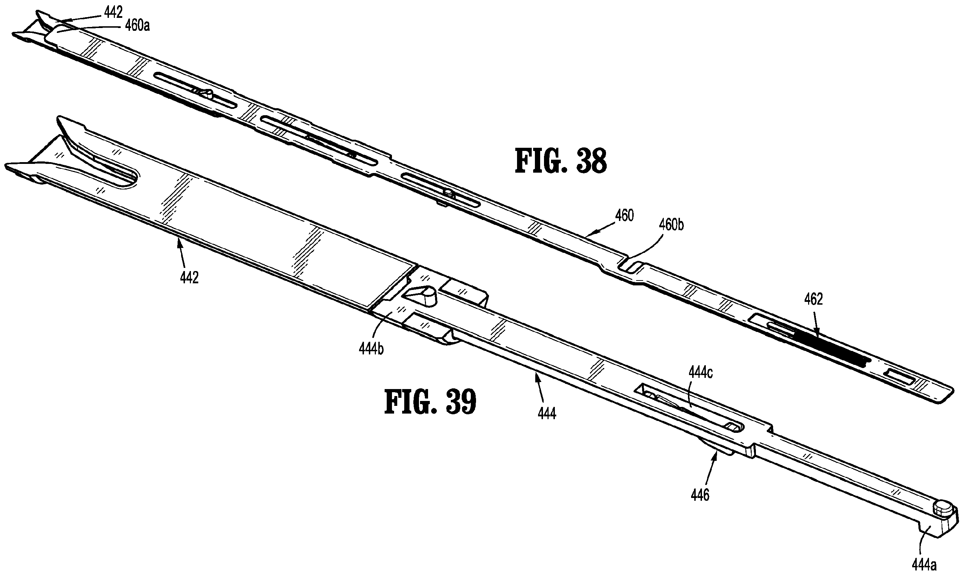

As illustrated in FIGS. 28 and 39, slider joint 444 is connected, at a proximal end 444a thereof, to a channel formed in spindle 440. A distal end 444b of slider joint 444 defines a substantially T-shaped profile, wherein the distal end 444b thereof is connected to jaw cam closure wedge 442. Latch member 446 functions as a linkage and is disposed to move through an aperture 444c in slider joint 444 to link with another fixed member and prevent slider joint 444 from advancing jaw cam closure wedge 442, and thus preventing the camming of jaw cam closure wedge 442 from camming the pair of jaws 450 to a closed condition during an initial stroke of trigger 104.

Spindle 440 is provided with a camming feature configured to move a cam link 448 (pivotably connected to a filler component 466, as will be described in greater detail below) a perpendicular manner relatively to a longitudinal axis of spindle 440 during a distal advancement of spindle 440.

Clip channel 470 of shaft assembly 420 slidably retains a stack of surgical clips "C" therein for application, in seriatim, to the desired tissue or vessel. A clip follower 472 is provided and slidably disposed within clip channel 470 at a location proximal of the stack of surgical clips "C". A biasing member 474 is provided to spring bias clip follower 472, and in turn, the stack of surgical clips "C", distally. A clip channel cover 476 is provided that overlies clip channel 470 to retain and guide clip follower 472, biasing member 474 and the stack of surgical clips "C" in clip channel 470.

As mentioned above, shaft assembly 420 includes a pusher bar 480 for loading a distal-most surgical clip "Cl" of the stack of surgical clips "C" into the pair of jaws 450. Pusher bar 480 includes a pusher 480a at a distal end thereof for engaging a backspan of the distal-most surgical clip "Cl" and urging the distal-most surgical clip "Cl" into the pair of jaws 450. Pusher bar 480 includes a fin or tab 480b extending therefrom and extending into a slot 482a of a trip block 482. Fin 480b of pusher bar 480 is acted upon by a biasing member (not shown) that is supported in trip block 482 to bias pusher bar 480 in a proximal direction.

In operation, in order for spindle 440 to advance pusher bar 480 during a distal movement thereof, spindle 440 supports a trip lever 484 and a biasing member 486 (e.g., leaf spring). During a distal movement of spindle 440, as illustrated in FIG. 31, a distal nose or tip 484a of trip lever 484 selectively engages pusher bar 480 to distally advance pusher bar 480 and load distal-most surgical clip "Cl" into the pair of jaws 450.

Also as mentioned above, shaft assembly 420 further includes a wedge plate 460 that is biased to a proximal position by a wedge plate spring 462. Wedge plate 460 is a flat bar shaped member having a number of windows formed therein. Wedge plate 460 has a distal-most position wherein a tip or nose of wedge plate 460 is inserted between the pair of jaws 450 to maintain the pair of jaws 450 in an open condition for loading of the distal-most surgical clip "Cl" therein. Wedge plate 460 has a proximal-most position, maintained by wedge plate spring 462, wherein the tip or nose of wedge plate 460 is retracted from between the pair of jaws 450.

As illustrated in FIGS. 28 and 38, wedge plate 460 defines a "U" or "C" shaped aperture or window 460b in a side edge thereof. The "C" shaped aperture or window 460b of wedge plate 460 selectively engages a cam link 448 supported on a filler plate 466. Cam link 448 selectively engages a surface of "C" shaped aperture or window 460b of wedge plate 460 to retain wedge plate 460 in a distal-most position such that a distal tip or nose 460a of wedge plate 460 is maintained inserted between the pair of jaws 450 to maintain the pair of jaws 450 splayed apart.

Shaft assembly 420 further includes a filler component 466 interposed between clip channel 470 and wedge plate 460, at a location proximal of the pair of jaws 450. Filler component 466 pivotably supports a cam link 448 that is engagable with wedge plate 460. In operation, during a distal advancement of spindle 440, a camming feature of spindle 440 engages a cam link boss of cam link 448 to thereby move cam link 448 out of engagement of wedge plate 460 and permit wedge plate 460 to return to the proximal-most position as a result of biasing member 462.

Trip block 482 defines an angled proximal surface 482b for engagement with a corresponding surface of trip lever 484 that will be discussed herein. As mentioned above, notch or slot 482a of trip block 482 is for receipt of fin 480b of pusher bar 480. In order to disengage trip lever 484 from a window 480c (FIG. 31) of pusher bar 480, and allow pusher bar 480 to return to a proximal-most position following loading of a surgical clip "C" into the pair of jaws 450, angled proximal surface 482b trip block 482 engages trip lever 484 to cam trip lever 484 out of window 480c of pusher bar 480. It is contemplated that spindle 440 may define a first cavity and a second cavity therein for receiving trip lever 484 and trip lever biasing spring 486, respectively. The first cavity may be provided with a pivoting boss to allow trip lever 484 to pivot between a first position and a second position. Trip lever biasing spring 486 may rest in the second cavity.

Trip lever biasing spring 486 functions to maintain a tip of trip lever 484 in contact with pusher bar 480, and more specifically, within window 480c of pusher bar 480 (FIG. 31) such that distal advancement of spindle 440 results in distal advancement of pusher bar 480, which in turn results in a loading of a distal-most surgical clip "Cl" in the pair of jaws 450.

With reference to FIGS. 28, 33 and 36, clip applier 10' also has a lockout bar 490. Lockout bar 490 includes a first end, and a second opposite hook end. The second hook end of lockout bar 490 is adapted to engage clip follower 472 of shaft assembly 420. Lockout bar 490 is pivotally retained in a slot formed in clip follower 472. Lockout bar 490 does not by itself lockout clip applier 10', but instead cooperates with the ratchet mechanism 150 of handle assembly 100 to lock out clip applier 10'.

Lockout bar 490 is adapted to move distally with clip follower 472 each time clip applier 10' is fired, and clip follower 472 is advanced distally. In operation, each time a surgical clip "C" is fired from clip applier 10', clip follower 472 will advance distally relative to the clip channel 470.

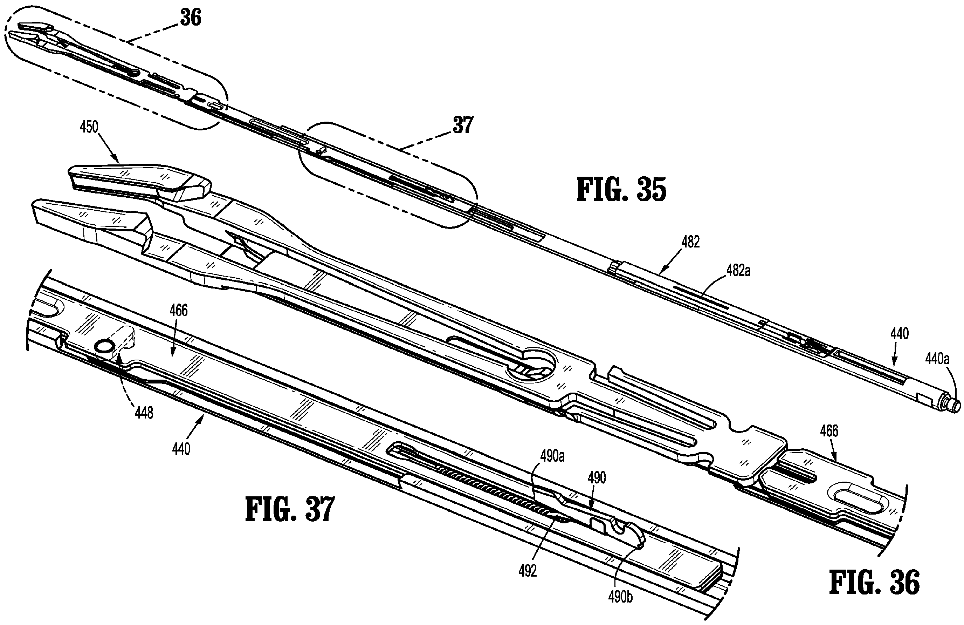

Pusher bar 480 defines a distal window therein (not shown). In operation, when clip follower 472 is positioned beneath pusher bar 480 (e.g., when there are no remaining surgical clips), a distal end 490a of lockout bar 490 will deflect upward (due to a biasing of a lockout biasing member 492), and enter a distal window 480d of pusher bar 480 to engage pusher bar 480 at a distal end of distal window 480d. Further, a proximal end 490b of lockout bar 490, defines a hook (FIG. 37), which is rotated into and engages an aperture defined in a floor of clip channel 470.

With the distal end of pusher bar 480 disposed within distal window 480d of pusher bar 480, pusher bar 480, and in turn, spindle 440 cannot return to a fully proximal position. Since spindle 440 cannot return to the fully proximal position, pawl 152 of ratchet mechanism 150 of handle assembly 100 cannot return to the home or initial position relative to rack 154 thereof. Instead, pawl 154 will remain in an intermediate position along rack 154, thus preventing trigger 104 from returning to a fully unactuated position.

With continued reference to FIGS. 26-29, an operation or firing of surgical clip applier 10', including endoscopic assembly 400 operatively connected to handle assembly 100, is shown and described. With endoscopic assembly 400 operatively connected to handle assembly 100, as trigger 104 of handle assembly 100 is actuated drive bar 104b of trigger 104 acts on drive plunger 120 to distally advance drive plunger 120. As trigger 104 is actuated, pawl 154 of ratchet assembly 150 begins to engage rack 152 thereof. With pawl 154 engaged with rack 152, trigger 104 may not return to a fully unactuated position until trigger 104 completes a full actuation or stroke thereof.

As drive plunger 120 is distally advanced, a distal end of drive plunger 120 presses against proximal wall 432b of cartridge cylinder 432 of drive assembly 430 of endoscopic assembly 400 to distally advance cartridge cylinder 432. Due to first spring constant "K1" of first biasing member 436 being larger or greater than second spring constant "K2" of second biasing member 438, as cartridge cylinder 432 is advanced distally, ring 435 acts on first biasing member 436 which in turn acts on fingers 434e of cartridge plunger 434 to push cartridge plunger 434 distally. As cartridge plunger 434 is distally advanced, cartridge plunger 434 distally advances inner shaft 424 relative to outer shaft 422. Being that second biasing member 438 is interposed between a flange 437a of collar 437 and proximal end 434b of cartridge plunger 434, as cartridge plunger 434 is distally advanced, cartridge plunger 434 also compresses second biasing member 438.

As inner shaft 424 is distally advanced relative to outer shaft 422, inner shaft 424 actuates a clip pusher (not shown) which in turn acts on a distal-most surgical clip (not shown) of a stack of surgical clips (not shown) to distally advance the distal-most surgical clip into the pair of jaws 450. Following loading of the distal-most surgical clip into the pair of jaws 450, the distal advancement of inner shaft 424 effects a closure of the pair of jaws 450 to form the surgical clip loaded therewithin.

When the pair of jaws 450 have fully closed to form the surgical clip loaded therein, or when the pair of jaws 450 have reached a hard stop, pawl 154 of ratchet assembly 150 of handle assembly 100 may not have cleared rack 152 thereof, and thus blocks or prevents trigger 104 from returning to a fully unactuated position thereof. Since the pair of jaws 450 cannot close any further, inner shaft 422 is also stopped from further distal advancement. However, as mentioned above, in order to return trigger 104 to the fully unactuated position, trigger 104 must first complete the full actuation stroke thereof. As such, as trigger 104 is further actuated to complete the full stroke thereof, as drive plunger 120 is continued to be driven distally, the distal end of drive plunger 120 continues to press against proximal wall 432b of cartridge cylinder 432 of drive assembly 430 of endoscopic assembly 400 to continue to distally advance cartridge cylinder 432.

With inner shaft 422, and in turn cartridge plunger 434, stopped from any further distal advancement, as cartridge cylinder 432 is continued to be advanced distally relative to cartridge plunger 434, cartridge cylinder 432 begins to and continues to compress first biasing member 436 until such time that pawl 154 of ratchet assembly 150 of handle assembly 100 clears and disengages rack 152 thereof. With pawl 154 of ratchet assembly 150 clear and disengaged from rack 152, trigger 104 may be released and returned to the fully unactuated position by hand, by a return spring (not shown) of trigger 104 or handle assembly 100 and/or by first biasing member 436 and second biasing member 438 of endoscopic assembly 400.

In accordance with the present disclosure, the trigger stroke length for trigger 104 of handle assembly 100 is constant or fixed, while the closure stroke length of the pair of jaws 450 of endoscopic assembly 400 connected to handle assembly 100 is different than, for example, the closure stroke of the pair of jaws 250 of endoscopic assembly 200. For example, endoscopic assembly 400 may require the pair of jaws 450 thereof to travel a relatively greater or smaller distance as compared to the pair of jaws 250 of endoscopic assembly 200 in order to complete a full opening and closing thereof. As such, universal handle assembly 100 may be loaded with, and is capable of firing, either endoscopic assembly 200 or endoscopic assembly 400.

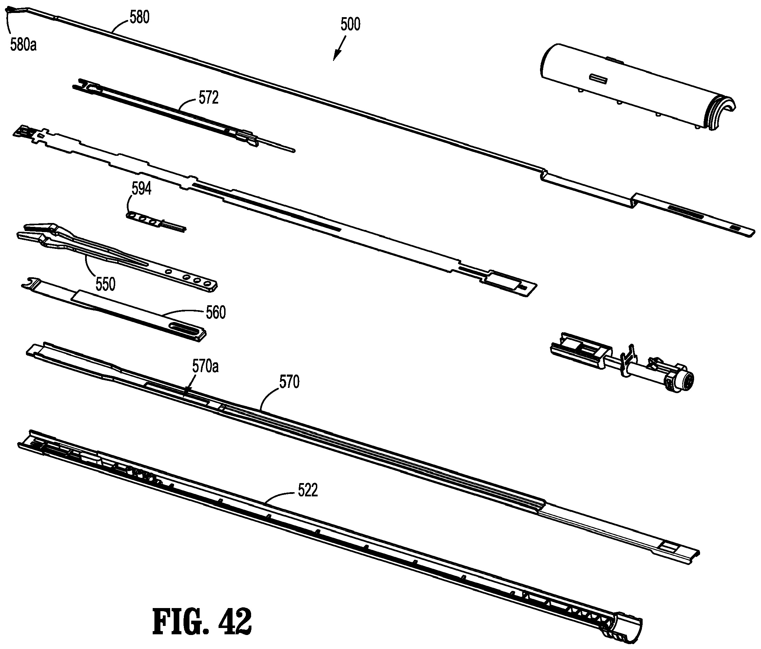

With reference to FIGS. 42-51, an alternate embodiment of the endoscopic assembly 400 is illustrated and generally identified by reference numeral 500. The endoscopic assembly 500 is substantially similar to that of the endoscopic assembly 400, and therefore, only the differences therebetween will be described in detail for purposes of brevity.

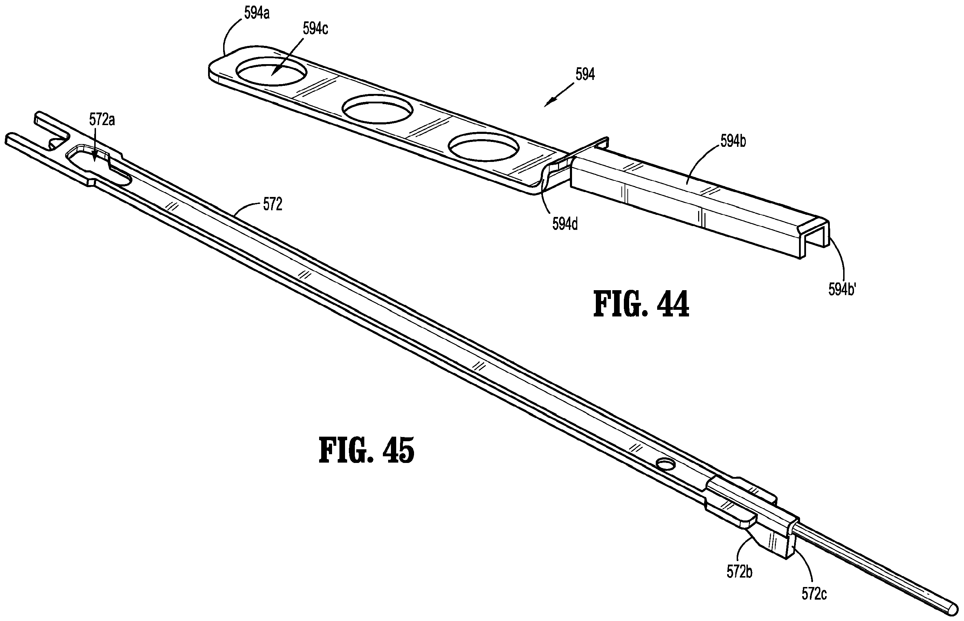

A distal portion of the clip follower 572 defines a window 572a (FIG. 45) that is configured to engage the pusher 580a of the pusher bar 580 after a last surgical clip "C" is formed, as will be described in further detail hereinbelow. A surface (e.g., bottom surface) of a proximal portion of the clip follower 572 defines a wedge or fin 572b that increases in thickness (e.g., extends further in a downward direction) in a proximal direction and terminates in a proximal surface 572c.

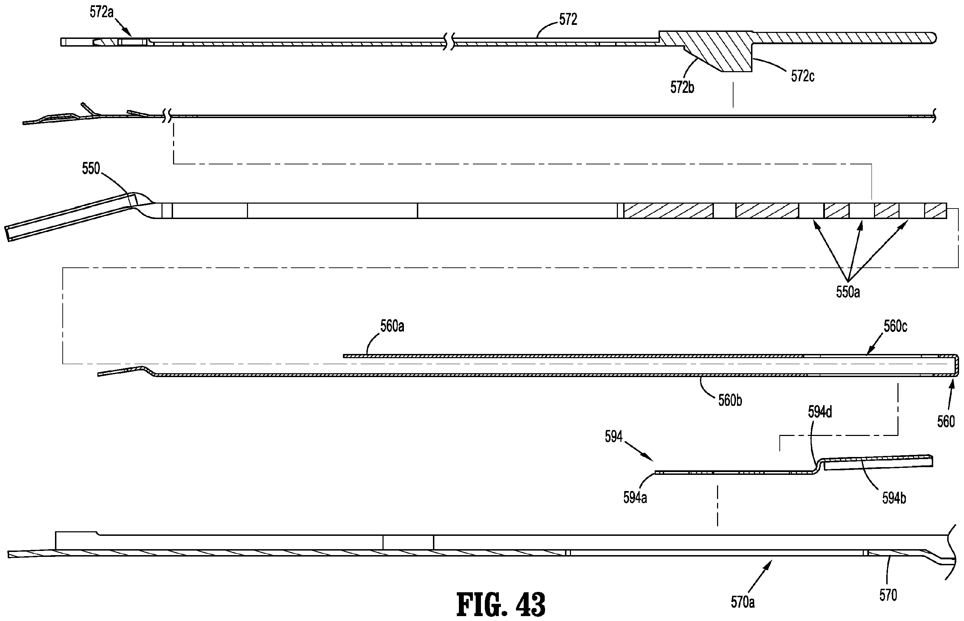

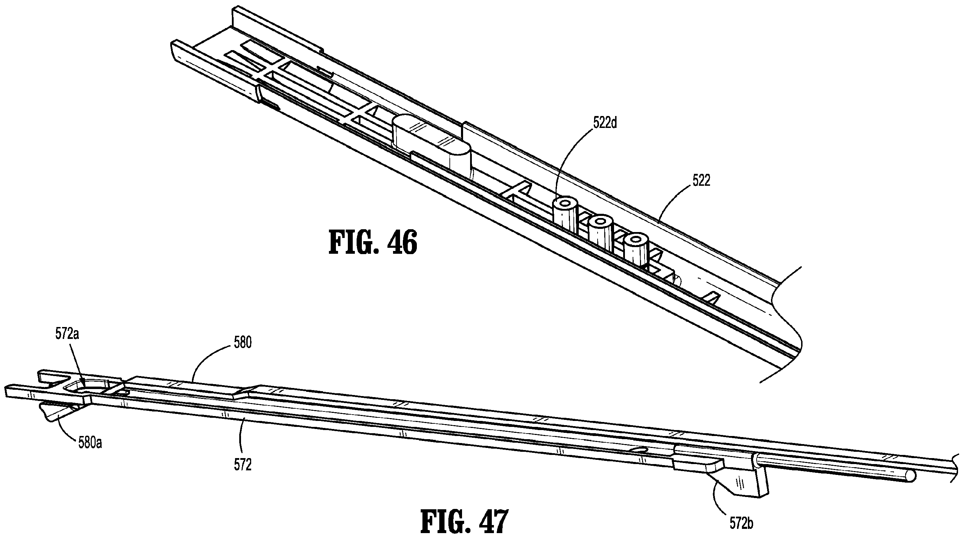

A proximal portion of the pair of jaws 550 defines a plurality of through-holes 550a (FIG. 43) therein which are configured to engage a corresponding plurality of shear pins 522d (FIG. 46) disposed on an interior portion of the outer tube 522. In this manner, when the plurality of shear pins 522d are received within the corresponding plurality of through-holes 550a, the shear pins 522d fixedly retain the pair of jaws 550 to the outer tube 522. Each shear pin, of the plurality of shear pins 522d, is configured to withstand a force equal to a first or threshold value. In this manner, when a force applied to the plurality of shear pins 522d exceeds the first or threshold value, the each shear pin, of the plurality of shear pins 522d, shears from the outer tube 522 (*e.g.,* breaks off the outer tube 522).

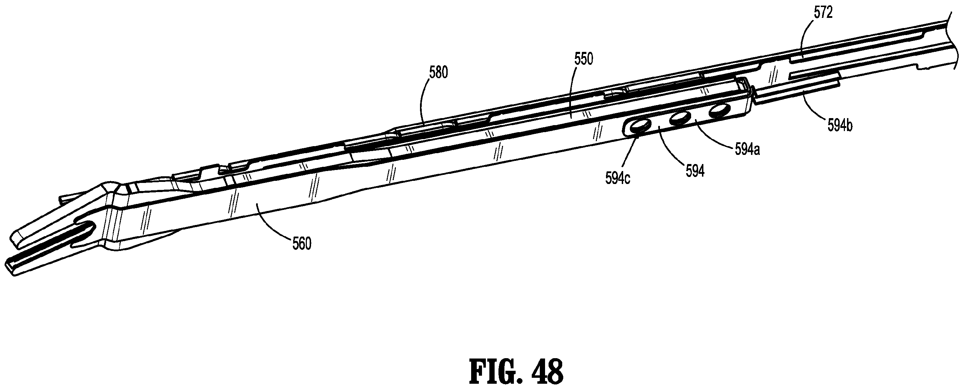

The endoscopic assembly 500 includes a wedge plate 560 having a generally U-shaped configuration and defining a pair of juxtaposed legs 560a and 560b (FIG. 43). The pair of legs 560a, 560b, of the wedge plate 560, is configured to receive a proximal portion of the pair of jaws 550 therein such that the proximal portion of the pair of jaws 550 is interposed between each leg 560a, 560b of the wedge plate 560 (FIG. 48). A proximal portion of the wedge plate 560 defines a slot 560c dimensioned to receive the plurality of shear pins 522d therethrough when the wedge plate 560, with the pair of jaws 550 received therein, is advanced over the plurality of shear pins 522d. In this manner, the slot 560c is defined through both legs of the pair of legs 560a, 560b of the wedge plate 560.

The endoscopic assembly 500 includes a lockout mechanism 594 having a distal elongate tab portion 594a and a proximal wedge portion 594b (FIG. 44). The tab portion 594a of the lockout mechanism 594 defines a generally rectangular configuration and is configured and dimensioned to be interposed between the wedge plate 560 and the spindle 540 (FIG. 48). The tab portion 594a of the lockout mechanism 594 defines a plurality of bores 594c therethrough. In this manner, each bore of the plurality of bores 594c includes a diameter dimensioned to receive a corresponding shear pin of the plurality of shear pins 522d, such that when the plurality of shear pins 522d are received within the plurality of bores 594c, the lockout mechanism 594 is maintained in fixed relation with respect to the pair of jaws 550.

The proximal wedge portion 594b of the lockout mechanism 594 defines a generally U-shaped (e.g., inverted) configuration and extends in an upward and proximal direction from the tab portion 594a. The proximal wedge portion 594b of the lockout mechanism 594 terminates in a proximal face 594b' and is coupled to the tab portion 594a at a distal portion by means of a resilient finger 594d. The resilient finger 594d of the lockout mechanism 594 acts as a living hinge such that tab portion 594b is permitted to remain stationary with respect to the pair of jaws 550 while the proximal wedge portion 594b is permitted to rotate relative to the tab portion 594b in a vertical orientation as the fin 572b is translated thereon, as will be described in further detail hereinbelow.

As can be appreciated, the lockout mechanism 594 may be monolithically formed or may be formed from one or more components defining the tab portion 594a, the proximal wedge portion 594b, and the resilient finger 594d. In embodiments, each of the tab portion 594a, proximal wedge portion 594b, and the resilient finger 594d may be formed from the same or different materials.

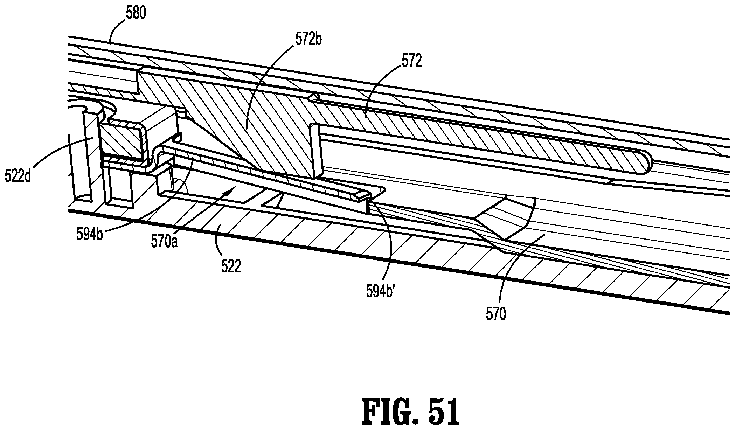

A spindle 570 of the endoscopic assembly 500 defines a slot 570a (FIG. 42) therethrough and is dimensioned to receive the plurality of shear pins 522d therein. In this manner, the slot 570a of the spindle 570 extends along the length thereof and includes sufficient length to accommodate the stroke of the spindle 570 as the spindle 570 is advanced in a distal direction. The slot 570a of the spindle 570 is further dimensioned such that when the proximal wedge portion 594b of the lockout mechanism 594 is depressed and fully received within the slot 570a (FIG. 51), the spindle 570 is inhibited from being advanced in a proximal direction (*e.g.,* a proximal portion of the proximal wedge portion 594b of the lockout mechanism 594 abuts a proximal wall of the slot 570a of the spindle 570), as will described in further detail hereinbelow.

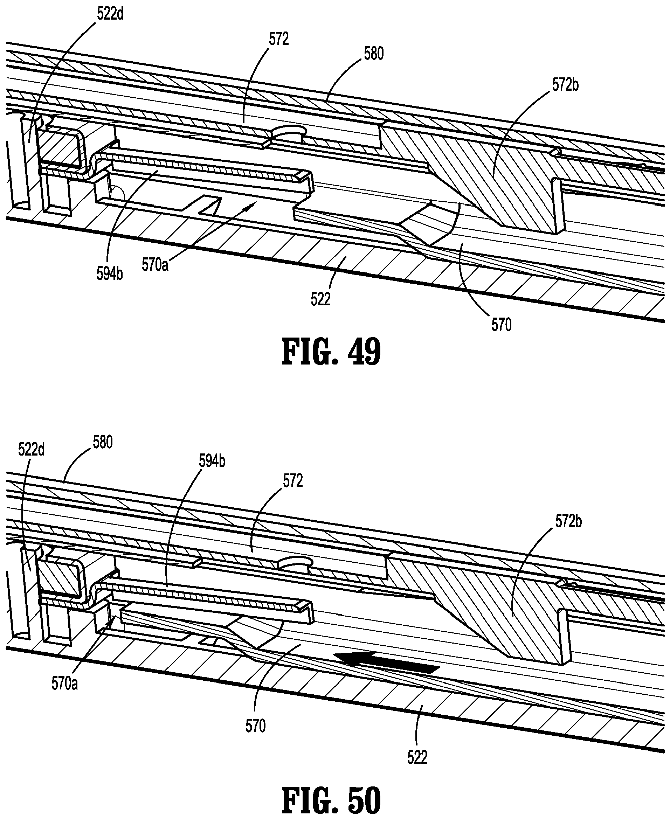

In use, during a firing of a surgical clip, the proximal wedge portion 594b of the lockout mechanism 594 is initially in a first, unactuated position, such that the spindle 570 is permitted to advance in a distal direction (FIG. 49). In this manner, the spindle 570 passes underneath the proximal wedge portion 594b (FIG. 50) of the lockout mechanism 594 to cause the pair of jaws 550 to approximate, and thereby form a surgical clip that has been loaded therein. After the firing of a final surgical clip, and the clip follower 572 is advanced in a distal position, the pusher 580a of the pusher bar 580 is received within the window 572a of the clip follower 572 (FIG. 47). Continued actuation of the trigger 104 of the handle assembly 100 causes the pusher 580a to further advance in a distal direction along with the clip follower 572. As the clip follower 572 is further advanced in a distal direction, the fin 572b of the clip follower 572 abuts the proximal wedge portion 594b of the lockout mechanism 594 which urges the proximal wedge portion 594b in a downward direction such that the proximal wedge portion 594b is received within the slot 570a of the spindle 570 (FIG. 51). Once received within the slot 570a of the spindle 570, the proximal face 594b' of the proximal wedge portion 594b abuts a proximal wall of the slot 570a and inhibits further distal advancement of the spindle 570 (FIG. 51). As can be appreciated, by inhibiting further distal advancement of the spindle 570, approximation of the pair of jaws 550 is likewise inhibited.

Continued actuation of the trigger 104 of the handle assembly 100 results in an increase in force applied to the spindle 570, the lockout mechanism 594, and the plurality of shear pins 522d. In this manner, the plurality of shear pins 522d provide a reaction force against the spindle 570 and prevent a clinician from being able to approximate the pair of jaws 550. If a clinician continues to actuate the trigger 104 of the handle assembly 100, the force applied to the plurality of shear pins 522d exceeds the first or threshold value and causes the plurality of shear pins 522d to shear (e.g., sever) from the outer tube 522 such that the pair of jaws 550 become decoupled from the outer tube 522. As can be appreciated, without the plurality of shear pins 522d retaining the pair of jaws 550 to the outer tube, continued distal advancement of the spindle 570 urges the pair of jaws 550 in a distal direction without causing the pair of jaws 550 to approximate (e.g., the pair of jaws 550 and the spindle 570 move in unison in a distal direction).

In accordance with the present disclosure, while the trigger stroke length of trigger 104 of handle assembly 100 is constant, the closure stroke length for the pair of jaws 250, 450 of each endoscopic assembly 200, 400 is unique for each respective endoscopic assembly 200, 400. Accordingly, each drive assembly 230, 430 of respective endoscopic assemblies 200, 400 functions to accommodate for the variations in the closure stroke lengths for the pair of jaws 250, 450 of respective endoscopic assemblies 200, 400.

To the extent consistent, handle assembly 100 and/or endoscopic assemblies 200, 400 may include any or all of the features of the handle assembly and/or endoscopic assemblies disclosed and described in International Patent Application No. PCT/CN2015/080845, filed June 5 2015, entitled "Endoscopic Reposable Surgical Clip Applier," International Patent Application No. PCT/CN2015/091603, filed on October 10, 2015, entitled "Endoscopic Surgical Clip Applier," and/or International Patent Application No. PCT/CN2015/093626, filed on November 3, 2015, entitled "Endoscopic Surgical Clip Applier," the entire content of each of which being incorporated herein by reference.

Surgical instruments such as the clip appliers described herein may also be configured to work with robotic surgical systems and what is commonly referred to as "Telesurgery." Such systems employ various robotic elements to assist the surgeon and allow remote operation (or partial remote operation) of surgical instrumentation. Various robotic arms, gears, cams, pulleys, electric and mechanical motors, etc. may be employed for this purpose and may be designed with a robotic surgical system to assist the surgeon during the course of an operation or treatment. Such robotic systems may include remotely steerable systems, automatically flexible surgical systems, remotely flexible surgical systems, remotely articulating surgical systems, wireless surgical systems, modular or selectively configurable remotely operated surgical systems, etc.

The robotic surgical systems may be employed with one or more consoles that are next to the operating theater or located in a remote location. In this instance, one team of surgeons or nurses may prep the patient for surgery and configure the robotic surgical system with one or more of the instruments disclosed herein while another surgeon (or group of surgeons) remotely control the instruments via the robotic surgical system. As can be appreciated, a highly skilled surgeon may perform multiple operations in multiple locations without leaving his/her remote console which can be both economically advantageous and a benefit to the patient or a series of patients.

The robotic arms of the surgical system are typically coupled to a pair of master handles by a controller. The handles can be moved by the surgeon to produce a corresponding movement of the working ends of any type of surgical instrument (e.g., end effectors, graspers, knifes, scissors, etc.) which may complement the use of one or more of the embodiments described herein. The movement of the master handles may be scaled so that the working ends have a corresponding movement that is different, smaller or larger, than the movement performed by the operating hands of the surgeon. The scale factor or gearing ratio may be adjustable so that the operator can control the resolution of the working ends of the surgical instrument(s).

The master handles may include various sensors to provide feedback to the surgeon relating to various tissue parameters or conditions, e.g., tissue resistance due to manipulation, cutting or otherwise treating, pressure by the instrument onto the tissue, tissue temperature, tissue impedance, etc. As can be appreciated, such sensors provide the surgeon with enhanced tactile feedback simulating actual operating conditions. The master handles may also include a variety of different actuators for delicate tissue manipulation or treatment further enhancing the surgeon's ability to mimic actual operating conditions.

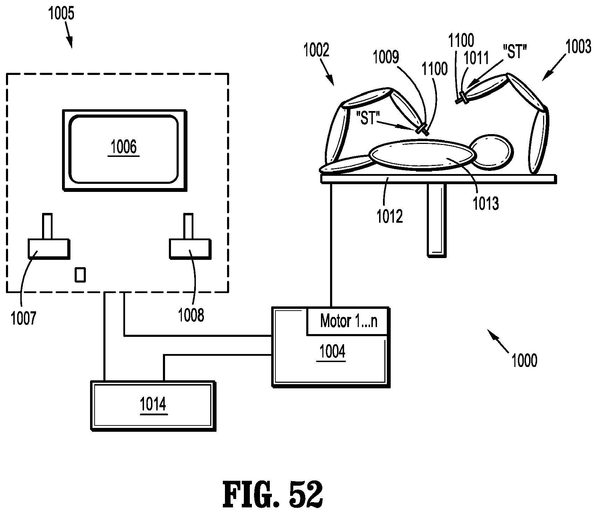

Referring to FIG. 52, a medical work station is shown generally as work station 1000 and generally may include a plurality of robot arms 1002, 1003; a control device 1004; and an operating console 1005 coupled with control device 1004. Operating console 1005 may include a display device 1006, which may be set up in particular to display three-dimensional images; and manual input devices 1007, 1008, by means of which a person (not shown), for example a surgeon, may be able to telemanipulate robot arms 1002, 1003 in a first operating mode.

Each of the robot arms 1002, 1003 may include a plurality of members, which are connected through joints, and an attaching device 1009, 1011, to which may be attached, for example, a surgical tool "ST" supporting an end effector 1100, in accordance with any one of several embodiments disclosed herein, as will be described in greater detail below.

Robot arms 1002, 1003 may be driven by electric drives (not shown) that are connected to control device 1004. Control device 1004 (e.g., a computer) may be set up to activate the drives, in particular by means of a computer program, in such a way that robot arms 1002, 1003, their attaching devices 1009, 1011 and thus the surgical tool (including end effector 1100) execute a desired movement according to a movement defined by means of manual input devices 1007, 1008. Control device 1004 may also be set up in such a way that it regulates the movement of robot arms 1002, 1003 and/or of the drives.

Medical work station 1000 may be configured for use on a patient 1013 lying on a patient table 1012 to be treated in a minimally invasive manner by means of end effector 1100. Medical work station 1000 may also include more than two robot arms 1002, 1003, the additional robot arms likewise being connected to control device 1004 and being telemanipulatable by means of operating console 1005. A medical instrument or surgical tool (including an end effector 1100) may also be attached to the additional robot arm. Medical work station 1000 may include a database 1014, in particular coupled to with control device 1004, in which are stored, for example, pre-operative data from patient/living being 1013 and/or anatomical atlases.

Reference is made herein to U.S. Patent Publication No. 2012/0116416, filed on November 3, 2011, entitled "Medical Workstation," the entire content of which is incorporated herein by reference, for a more detailed discussion of the construction and operation of an exemplary robotic surgical system.

It is contemplated, and within the scope of the present disclosure, that other endoscopic assemblies, including a pair of jaws having a unique and diverse closure stroke length thereof, may be provided with a drive assembly, similar to any of the drive assemblies described herein, for accommodating and adapting the closure stroke length for the pair of jaws thereof to the constant trigger stroke length.

Accordingly, various endoscopic assemblies, constructed in accordance with the principles of the present disclosure, may be provided which are also capable of firing or forming or closing surgical clips of various sizes, materials, and configurations, across multiple platforms for multiple different manufactures.

It should be understood that the foregoing description is only illustrative of the present disclosure. Various alternatives and modifications can be devised by those skilled in the art without departing from the disclosure. Accordingly, the present disclosure is intended to embrace all such alternatives, modifications and variances. The embodiments described with reference to the attached drawing figures are presented only to demonstrate certain examples of the disclosure. Other elements, steps, methods and techniques that are insubstantially different from those described above and/or in the appended claims are also intended to be within the scope of the disclosure.

The invention may be described by reference to the following numbered paragraphs:-
1. A reposable surgical clip applier, comprising:
   a handle assembly; and
   an endoscopic assembly selectively connectable to and in mechanical communication with the handle assembly, the endoscopic assembly including a shaft assembly having:
      a pair of jaws pivotably and fixedly supported in, and extending from a distal portion of the shaft assembly;
      a spindle slidably supported within the endoscopic assembly, the spindle including a proximal end and a distal end, the distal end of the spindle being operatively engaged with the pair of jaws to effectuate an opening and a closing of the pair of jaws upon an axial translation of the spindle relative to pair of jaws; and
      a lockout mechanism fixedly supported on the pair of jaws, the lockout mechanism being in selective engagement with the spindle, wherein the lockout mechanism includes a first position that enables distal advancement of the spindle and a second position that inhibits distal advancement of the spindle.
2. The reposable surgical clip applier according to paragraph 1, wherein the shaft assembly further includes a pusher bar slidably supported within the endoscopic assembly.
3. The reposable surgical slip applier according to paragraph 2, wherein the shaft assembly further includes a clip follower slidably disposed in the endoscopic assembly, the clip follower interposed between the pusher bar and the pair of jaws.
4. The reposable surgical clip applier according to paragraph 3, wherein a distal portion of the clip follower defines a window therethrough, the window being configured to receive a pusher defined on a distal portion of the pusher bar.
5. The reposable surgical clip applier according to paragraph 4, wherein a proximal portion of the clip follower defines a wedge extending proximally and outwardly therefrom, the wedge configured to selectively engage a portion of the lockout mechanism.
6. The reposable surgical clip applier according to paragraph 5, wherein the lockout mechanism defines a distal elongate tab portion and a proximal wedge portion, the distal elongate tab portion configured to be interposed between the pair of jaws and the spindle, the proximal wedge portion configured to selectively engage the wedge of the clip follower as the wedge of the clip follower is advanced over the proximal wedge portion.
7. The reposable surgical clip applier according to paragraph 6, wherein the lockout mechanism includes a resilient finger that couples the proximal wedge portion to the distal elongate tab portion such that the proximal wedge portion is free to rotate relative to the distal elongate tab portion.
8. The reposable surgical clip applier according to paragraph 7, wherein the spindle defines a window therethrough configured to receive the proximal wedge portion of the lockout mechanism, wherein, when the proximal wedge portion of the lockout mechanism is disposed in the window of the spindle, further distal advancement of the spindle is inhibited.
9. The reposable surgical clip applier according to paragraph 1, wherein the shaft assembly defines a plurality of shear pins on an interior surface thereof.
10. The reposable surgical clip applier according to paragraph 9, wherein the pair of jaws and the lockout mechanism define a plurality of through holes configured to receive the plurality of shear pins.
11. The reposable surgical clip applier according to paragraph 10, wherein the shear pins are configured to shear from the shaft assembly when a force exceeding a threshold value is applied to the shear pins.
12. The reposable surgical clip applier according to paragraph 7, wherein the resilient finger is configured to bias the proximal wedge portion of the lockout mechanism in an upward direction with respect to the distal elongate tab portion of the lockout mechanism.
13. A lockout assembly for use with a reposable surgical clip applier, comprising:
   a clip follower having a proximal portion and a distal portion, the distal portion configured to engage a pusher bar of a reposable surgical clip applier; and
   a lockout mechanism including a distal elongate tab portion, a proximal wedge portion, and a resilient finger interposed between the distal elongate tab portion and the proximal wedge portion to couple the distal elongate tab portion to the proximal wedge portion,
   wherein a proximal portion of the clip follower is configured to selectively engage the proximal wedge portion of the lockout mechanism such that when the proximal wedge portion of the lockout mechanism is engaged by the proximal portion of the clip follower, the proximal wedge portion configured to inhibit closure of a pair of jaws of a reposable surgical clip applier.
14. The lockout assembly for use with a reposable surgical clip applier according to paragraph 13, wherein the distal portion of the clip follower defines a window therethrough configured to receive a portion of a pusher of a reposable surgical clip applier.
15. The lockout assembly for use with a reposable surgical clip applier according to paragraph 14, wherein the proximal portion of the clip follower defines a wedge extending proximally and outwardly therefrom, the wedge being configured to selectively engage the proximal wedge portion of the lockout mechanism as the wedge is advanced over the proximal wedge portion of the lockout mechanism.
16. The lockout assembly for use with a reposable surgical clip applier according to paragraph 15, wherein when the proximal wedge portion is in a first position, a pair of jaws of a reposable surgical clip applier is permitted to close, and when the proximal wedge portion is in a second position, a pair of jaws of a reposable surgical clip applier is inhibited from closing.
17. The lockout assembly for use with a reposable surgical clip applier according to paragraph 16, wherein the proximal wedge portion defines a proximal face configured to engage a spindle of a reposable surgical clip applier to inhibit closure of a pair of jaws of a reposable surgical clip applier.
18. The lockout assembly for use with a reposable surgical clip applier according to paragraph 17, wherein the lockout mechanism defines a plurality of bores configured to receive a corresponding plurality of shear pins disposed on a reposable surgical clip applier.
19. The lockout assembly for use with a reposable surgical clip applier according to paragraph 18, wherein the proximal wedge portion of the lockout mechanism defines a U-shaped configuration.
20. The lockout assembly for use with a reposable surgical clip applier according to paragraph 13, wherein the lockout mechanism is maintained in fixed relation to a pair of jaws of a reposable surgical clip applier.

## Claims

1. A reposable surgical clip applier, comprising:
a handle assembly; and
an endoscopic assembly selectively connectable to and in mechanical communication with the handle assembly, the endoscopic assembly including a shaft assembly having:
a pair of jaws pivotably and fixedly supported in, and extending from a distal portion of the shaft assembly;
a spindle slidably supported within the endoscopic assembly, the spindle including a proximal end and a distal end, the distal end of the spindle being operatively engaged with the pair of jaws to effectuate an opening and a closing of the pair of jaws upon an axial translation of the spindle relative to pair of jaws; and
a lockout mechanism fixedly supported on the pair of jaws, the lockout mechanism being in selective engagement with the spindle, wherein the lockout mechanism includes a first position that enables distal advancement of the spindle and a second position that inhibits distal advancement of the spindle.

2. The reposable surgical clip applier according to claim 1, wherein the shaft assembly further includes a pusher bar slidably supported within the endoscopic assembly; preferably wherein the shaft assembly further includes a clip follower slidably disposed in the endoscopic assembly, the clip follower interposed between the pusher bar and the pair of jaws; preferably still wherein a distal portion of the clip follower defines a window therethrough, the window being configured to receive a pusher defined on a distal portion of the pusher bar.

3. The reposable surgical clip applier according to claim 2, wherein a proximal portion of the clip follower defines a wedge extending proximally and outwardly therefrom, the wedge configured to selectively engage a portion of the lockout mechanism.

4. The reposable surgical clip applier according to claim 3, wherein the lockout mechanism defines a distal elongate tab portion and a proximal wedge portion, the distal elongate tab portion configured to be interposed between the pair of jaws and the spindle, the proximal wedge portion configured to selectively engage the wedge of the clip follower as the wedge of the clip follower is advanced over the proximal wedge portion; preferably wherein the lockout mechanism includes a resilient finger that couples the proximal wedge portion to the distal elongate tab portion such that the proximal wedge portion is free to rotate relative to the distal elongate tab portion; preferably still wherein the spindle defines a window therethrough configured to receive the proximal wedge portion of the lockout mechanism, wherein, when the proximal wedge portion of the lockout mechanism is disposed in the window of the spindle, further distal advancement of the spindle is inhibited.

5. The reposable surgical clip applier according to any preceding claim, wherein the shaft assembly defines a plurality of shear pins on an interior surface thereof.

6. The reposable surgical clip applier according to claim 5, wherein the pair of jaws and the lockout mechanism define a plurality of through holes configured to receive the plurality of shear pins.

7. The reposable surgical clip applier according to claim 6, wherein the shear pins are configured to shear from the shaft assembly when a force exceeding a threshold value is applied to the shear pins.

8. The reposable surgical clip applier according to any preceding claim, wherein the resilient finger is configured to bias the proximal wedge portion of the lockout mechanism in an upward direction with respect to the distal elongate tab portion of the lockout mechanism.

9. A lockout assembly for use with a reposable surgical clip applier, comprising:
a clip follower having a proximal portion and a distal portion, the distal portion configured to engage a pusher bar of a reposable surgical clip applier; and
a lockout mechanism including a distal elongate tab portion, a proximal wedge portion, and a resilient finger interposed between the distal elongate tab portion and the proximal wedge portion to couple the distal elongate tab portion to the proximal wedge portion,
wherein a proximal portion of the clip follower is configured to selectively engage the proximal wedge portion of the lockout mechanism such that when the proximal wedge portion of the lockout mechanism is engaged by the proximal portion of the clip follower, the proximal wedge portion configured to inhibit closure of a pair of jaws of a reposable surgical clip applier.

10. The lockout assembly for use with a reposable surgical clip applier according to claim 9, wherein the distal portion of the clip follower defines a window therethrough configured to receive a portion of a pusher of a reposable surgical clip applier.

11. The lockout assembly for use with a reposable surgical clip applier according to claim 14, wherein the proximal portion of the clip follower defines a wedge extending proximally and outwardly therefrom, the wedge being configured to selectively engage the proximal wedge portion of the lockout mechanism as the wedge is advanced over the proximal wedge portion of the lockout mechanism.

12. The lockout assembly for use with a reposable surgical clip applier according to claim 11, wherein when the proximal wedge portion is in a first position, a pair of jaws of a reposable surgical clip applier is permitted to close, and when the proximal wedge portion is in a second position, a pair of jaws of a reposable surgical clip applier is inhibited from closing.

13. The lockout assembly for use with a reposable surgical clip applier according to claim 11 or claim 12, wherein the proximal wedge portion defines a proximal face configured to engage a spindle of a reposable surgical clip applier to inhibit closure of a pair of jaws of a reposable surgical clip applier.

14. The lockout assembly for use with a reposable surgical clip applier according to claim 13, wherein the lockout mechanism defines a plurality of bores configured to receive a corresponding plurality of shear pins disposed on a reposable surgical clip applier; preferably wherein the proximal wedge portion of the lockout mechanism defines a U-shaped configuration.

15. The lockout assembly for use with a reposable surgical clip applier according to any of claims 9 to 14, wherein the lockout mechanism is maintained in fixed relation to a pair of jaws of a reposable surgical clip applier.
